(19) Europäisches Patentamt
European Patent Office
Office européen des brevets

(11) Veröffentlichungsnummer: **0 054 715**
**A2**

(12) # EUROPÄISCHE PATENTANMELDUNG

(21) Anmeldenummer: **81109203.0**

(22) Anmeldetag: **29.10.81**

(51) Int. Cl.³: **C 07 C 103/38**, A 01 N 39/02, A 01 N 41/06, A 01 N 57/20, C 07 C 83/08, C 07 C 103/32, C 07 C 131/00, C 07 C 131/02, C 07 C 131/04, C 07 C 143/74, C 07 D 207/404

(30) Priorität: **18.12.80 CH 9345/80**
**04.09.81 CH 5722/81**

(43) Veröffentlichungstag der Anmeldung: **30.06.82**
**Patentblatt 82/26**

(84) Benannte Vertragsstaaten: **AT BE CH DE FR IT LI NL SE**

(71) Anmelder: **F. HOFFMANN-LA ROCHE & CO.**
**Aktiengesellschaft, CH-4002 Basel (CH)**

(72) Erfinder: **Fráter, Georg, Dr., Am Pfisterhölzli 22,**
**CH-8606 Greifensee (CH)**
Erfinder: **Suchy, Milos, Dr., Grossplatzstrasse 3,**
**CH-8122 Pfaffhausen (CH)**
Erfinder: **Winternitz, Paul, Dr., Am Pfisterhölzli 50,**
**CH-8606 Greifensee (CH)**

(74) Vertreter: **Lederer, Franz, Dr. et al, Patentanwälte Dr.**
**Lederer Franz Meyer-Roxlau Reiner F.**
**Lucile-Grahn-Strasse 22, D-8000 München 80 (DE)**

(54) **Propionsäureester, Herstellung dieser Verbindungen, Unkrautbekämpfungsmittel, die diese Verbindungen als Wirkstoffe enthalten, sowie Verwendung solcher Verbindungen und Mittel zur Bekämpfung von Unkräutern.**

(57) Propionsäureester der Formel

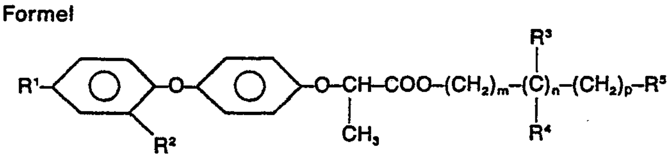

worin

$R^1$ Chlor, Brom, Jod oder Trifluoromethyl

$R^2$ Wasserstoff, Chlor, Brom oder Jod

$R^3$ Wasserstoff oder $C_{1-4}$-Alkyl

$R^4$ Wasserstoff; $C_{1-4}$-Alkyl; oder Phenyl, gegebenenfalls substituiert durch Halogen, $C_{1-4}$-Alkyl und/oder Nitro, wobei $R_3$ und $R_4$ zusammen mit dem Kohlenstoffatom, an das sie geknüpft sind, auch einen $C_{5-8}$-Cycloalkanring bilden können, der gegebenenfalls durch 1 bis 3 $C_{1-4}$-Alkylgruppen substituiert werden kann, m, n und p die Zahl 0 oder 1 und $R_5$ mehrere Bedeutungen haben kann, Verfahren zu deren Herstellung, Unkrautbekämpfungsmittel, die diese Verbindungen als Wirkstoffe enthalten, sowie die Verwendung solcher Verbindungen bzw. Mittel zur Bekämpfung von Unkräutern.

**EP 0 054 715 A2**

0054715

F.Hoffmann-La Roche & Co.Aktiengesellschaft, Basel/Schweiz

RAN 6102/27

# Propionsäureester, Herstellung dieser Verbindungen, Unkrautbekämpfungsmittel, die diese Verbindungen als Wirkstoffe enthalten, sowie Verwendung solcher Verbindungen und Mittel zur Bekämpfung von Unkräutern.

Die Erfindung betrifft Propionsäureester der allgemeinen Formel

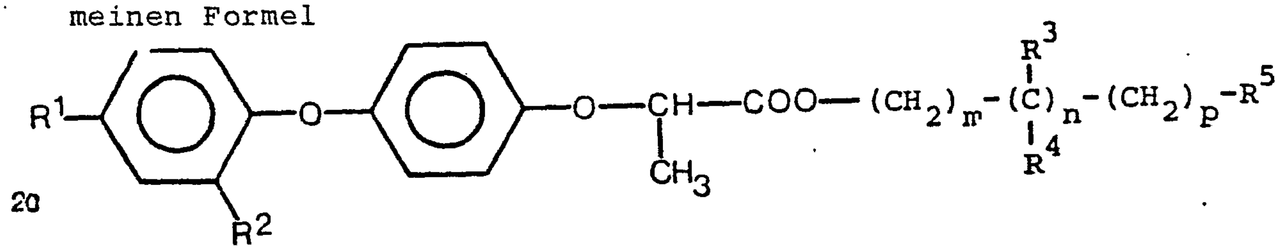

I

worin $R^1$ Chlor, Brom, Jod oder Trifluormethyl,

$R^2$ Wasserstoff, Chlor, Brom oder Jod,

$R^3$ Wasserstoff oder $C_{1-4}$-Alkyl,

$R^4$ Wasserstoff; $C_{1-4}$-Alkyl; oder Phenyl, gegebenenfalls substituiert mit Halogen, $C_{1-4}$-Alkyl und/oder Nitro,

wobei $R^3$ und $R^4$ zusammen mit dem Kohlenstoffatom, an das sie geknüpft sind, auch einen $C_{5-8}$-Cycloalkanring bilden können, der gegebenenfalls mit 1 bis 3 $C_{1-4}$-Alkylgruppen substituiert sein kann,

m, n und p die Zahl 0 oder 1

und $R^5$ eine der folgenden Gruppen (a) - (f) bedeuten:

Pa/19.10.81

$-NHCO-R^6$ (a)

$-NHSO_2-R^7$ (b)

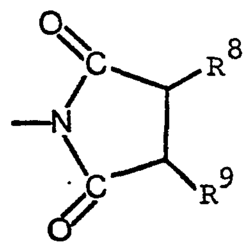 (c)

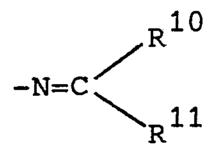 (d)

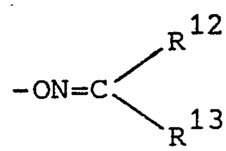 (e)

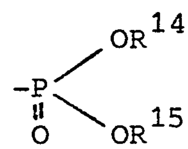 (f)

wobei in den obigen Formeln der Gruppen (a) - (f)

$R^6$ Wasserstoff; $C_{1-6}$-Alkyl; $C_{1-4}$-Halogenalkyl; oder Phenyl, gegebenenfalls substituiert mit Halogen, $C_{1-4}$-Alkyl und/oder Nitro bedeutet,

$R^7$ $C_{1-6}$-Alkyl; oder Phenyl, gegebenenfalls substituiert mit Halogen, $C_{1-4}$-Alkyl und/oder Nitro bedeutet,

$R^8$ und $R^9$ für Wasserstoff stehen oder zusammen eine Bindung, Trimethylen, Tetramethylen, 2-Butenylen oder einen ankondensierten Benzolring bedeuten,

$R^{10}$ $C_{1-6}$-Alkyl und $R^{11}$ Trifluormethyl, $C_{1-6}$-Alkoxy, $C_{1-6}$-Alkoxy-$C_{1-4}$-alkyl, $C_{2-7}$-Alkanoyl, $C_{2-7}$-Alkoxycarbonyl oder $C_{2-7}$-Alkoxycarbonyl-$C_{1-4}$-alkyl bedeuten, oder $R^{10}$ und $R^{11}$ zusammen mit dem Kohlenstoffatom, an das sie geknüpft sind, einen Cyclopentanring bilden, der gegebenenfalls mit 1 bis 3 $C_{1-4}$-Alkyl und/oder 1 $C_{1-4}$-Alkoxy sub-

stituiert sein kann, wobei, falls $R^{10}$, $R^{11}$ und das Kohlenstoffatom einen gegebenenfalls substituierten Cyclopentanring bilden, $R^1$ Chlor, Brom oder Trifluormethyl ist,

$R^{12}$ Wasserstoff oder $C_{1-6}$-Alkyl und $R^{13}$ $C_{1-6}$-Alkyl; oder Phenyl, gegebenenfalls substituiert mit Halogen, $C_{1-4}$-Alkyl und/oder Nitro bedeuten, oder $R^{12}$ und $R^{13}$ zusammen mit dem Kohlenstoffatom, an das sie geknüpft sind, einen $C_{5-7}$-Cycloalkanring bilden, und

$R^{14}$ und $R^{15}$ $C_{1-6}$-Alkyl oder Phenyl bedeuten, und wobei, wenn $R^5$ eine Gruppe (a), (b) oder (c) darstellt, m die Zahl 1, n die Zahl 0 und p die Zahl 0 oder 1 bedeuten; wenn $R^5$ eine Gruppe (d) darstellt, m, n und p die Zahl 0 bedeuten; wenn $R^5$ eine Gruppe (e) darstellt, m die Zahl 0 und n und p die Zahl 1 bedeuten; und wenn $R^5$ eine Gruppe (f) darstellt, m und p die Zahl 0 oder 1 und n die Zahl 1 bedeuten.

Die Verbindungen der Formel I besitzen herbizide Eigenschaften und eignen sich somit als (Wirkstoffe von) Unkrautbekämpfungsmittel(n). Somit umfasst die Erfindung auch Unkrautbekämpfungsmittel, die mindestens eine Verbindung der Formel I als Wirkstoff enthalten, ein Verfahren zur Herstellung dieser Verbindungen sowie die Verwendung solcher Verbindungen bzw. Mittel zur Bekämpfung von Unkräutern.

In der obigen Formel I umfasst der Ausdruck "Halogen" Fluor, Chlor, Brom und Jod. Unter "$C_{1-4}$-Alkyl" bzw. "$C_{1-6}$-Alkyl" sind sowohl geradkettige als auch verzweigte Alkylgruppen zu verstehen, wie Methyl, Aethyl, n-Propyl, Isopropyl, n-Butyl, Isobutyl, tert.Butyl, n-Pentyl, Isoamyl, neo-Pentyl und n-Hexyl. Dies gilt auch für $C_{1-4}$-Alkylgruppen bzw. $C_{1-6}$-Alkylgruppen enthaltende Reste, wie $C_{1-4}$- bzw. $C_{1-6}$-Alkoxy, $C_{2-7}$-Alkoxycarbonyl, $C_{1-4}$-Halogenalkyl, $C_{2-7}$-Alkoxycarbonyl-$C_{1-4}$-alkyl, $C_{1-6}$-Alkoxy-$C_{1-4}$-alkyl und $C_{2-7}$-Alkanoyl. Wenn in einem substituierten Phenylrest zwei oder mehr Substituenten vorhanden sind, können die Substituenten gleich oder verschieden sein. Dies gilt auch für den mit Alkyl substituierten $C_{5-8}$-Cycloalkanring und den mit Alkyl und/oder Alkoxy substituierten Cyclopentanring. Der Ausdruck "Halogenalkyl" umfasst mit einem oder mehreren Halogenatomen substituiertes Alkyl.

Da mindestens ein asymmetrisches Kohlenstoffatom im Molekül vorhanden ist, können die Verbindungen der Formel I in optisch aktiver Form auftreten. Durch das Vorliegen der Stickstoff-Kohlenstoff-Doppelbindung in den Verbindungen der Formel I, worin $R^5$ eine Gruppe (d) oder eine Gruppe (e) bedeutet, tritt für jene Verbindungen, worin $R^{10}$ und $R^{11}$ bzw. $R^{12}$ und $R^{13}$ unterschiedliche Bedeutung haben, zusätzlich geometrische Isomerie auf. Solche geometrischen Isomeren werden als syn- und anti-Formen bezeichnet. Unabhängig davon kommt auch in gewissen Fällen eine Atropisomerie vor. Die Formel I soll demnach die Racemate sowie all diese möglichen isomeren Formen umfassen.

Unabhängig voneinander bedeuten $R^1$ vorzugsweise Jod oder Trifluormethyl und $R^2$ vorzugsweise Wasserstoff oder Chlor.

$R^3$ in der Bedeutung $C_{1-4}$-Alkyl ist vorzugsweise Methyl.

$R^6$ bedeutet vorzugsweise $C_{1-6}$-Alkyl.

$R^7$ bedeutet vorzugsweise gegebenenfalls substituiertes Phenyl.

$R^8$ und $R^9$ bedeuten beide vorzugsweise Wasserstoff.

Besonders bevorzugt sind die D-Formen der Verbindungen der Formel I.

Bevorzugte Verbindungen der Formel I sind:

D-2-[p-(α,α,α-Trifluor-p-tolyloxy)phenoxy]-propionsäure-succinimidomethylester,

Aethyl-N-/D-2-[p-(α,α,α-trifluor-p-tolyloxy)phenoxy]-propionoxy/-acetimidat,

n-Butyl-N-/D-2-[p-(α,α,α-trifluor-p-tolyloxy)phenoxy]-propionoxy/-acetimidat,

D-2-[p-(α,α,α-Trifluor-p-tolyloxy)phenoxy]-propionsäure-2-isopropylidenaminooxy-äthylester und

D-2-[p-(α,α,α-Trifluor-p-tolyloxy)phenoxy]-propionsäure-diäthoxyphosphinylmethylester.

Das erfindungsgemässe Verfahren zur Herstellung der Verbindungen der Formel I ist dadurch gekennzeichnet, dass man

a) eine Säure der Formel

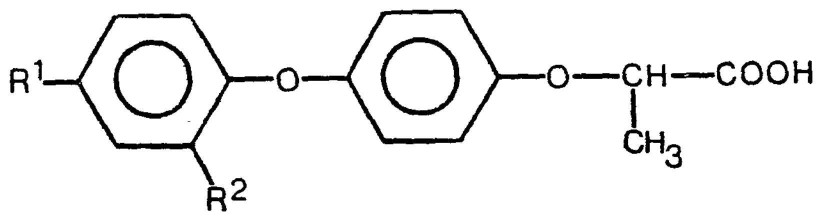

II

worin $R^1$ und $R^2$ die oben angegebenen Bedeutungen besitzen,

oder ein reaktionsfähiges Derivat davon, mit einer Verbindung der Formel

$$X-(CH_2)_m-(\overset{R^3}{\underset{R^4}{C}})_n-(CH_2)_p-R^5 \qquad III$$

worin $R^3$, $R^4$, $R^5$, m, n und p die oben angegebenen Bedeutungen besitzen und X eine Abgangsgruppe bedeutet,

umsetzt, oder

b) ein Phenol der Formel

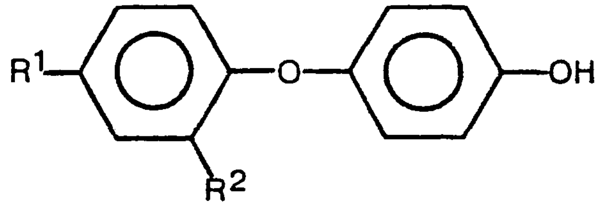 IV

worin $R^1$ und $R^2$ die oben angegebenen Bedeutungen besitzen,

oder ein Alkalimetallsalz davon, mit einer Verbindung der Formel

$$Y-\underset{CH_3}{\underset{|}{CH}}-COO-(CH_2)_m-(\overset{R^3}{\underset{R^4}{C}})_n-(CH_2)_p-R^5 \qquad V$$

worin $R^3$, $R^4$, $R^5$, m, n und p die oben angegebenen Bedeutungen besitzen und Y eine Abgangsgruppe bedeutet,

umsetzt.

Bei der Verfahrensvariante a) handelt es sich um eine Veresterung, die nach an sich bekannten Methoden durchgeführt werden kann, z.B. gemäss den nachfolgenden Varianten $a_1$), $a_2$) und $a_3$):

| | Edukt | Reagens |
|---|---|---|
| $a_1$) | Freie Säure der Formel II, insbesondere aber Salze davon | Verbindung der Formel III, worin X beispielsweise für Chlor, Brom, Jod, Mesyloxy oder Tosyloxy steht |
| $a_2$) | Halogenid, Imidazolid oder Anhydrid der Säure der Formel II | Verbindung der Formel III, worin X für Hydroxy steht |
| $a_3$) | Freie Säure der Formel II | Verbindung der Formel III, worin X für Hydroxy steht (III insbesondere = Oxim) |

Bei der Verfahrensvariante $a_1$) bedeutet der Ausdruck "Salz" beispielsweise ein Alkalimetallsalz, z.B. das Natrium-, Kalium- oder Lithiumsalz, ein Erdalkalimetallsalz, z.B. das Magnesium-, Calcium- oder Bariumsalz, ein Salz einer organischen Base, z.B. ein Mono-, Di- oder Trialkylammoniumsalz oder ein Pyridiniumsalz, oder das Ammoniumsalz. Der Ausdruck "Abgangsgruppe" für X steht vorzugsweise für Chlor, Brom, Jod, Mesyloxy oder Tosyloxy.

Die Veresterung der Säure der Formel II, vorzugsweise in Form eines Salzes, mit der Verbindung der Formel III wird vorzugsweise in einem inerten Lösungsmittel und bei Temperaturen von etwa -20°C bis 100°C durchgeführt. Ein besonders bevorzugter Temperaturbereich ist derjenige zwischen 0°C und 40°C. Es kommen als Lösungsmittel vorzugsweise inerte organische Lösungsmittel in Frage. Hierzu gehören vorzugsweise Aether, z.B. Diäthyläther und Tetrahydrofuran, chlorierte Kohlenwasserstoffe, z.B. Dichlormethan und Chloroform, und Dimethylformamid.

Bei Verwendung der freien Säure der Formel II wird die Umsetzung zweckmässigerweise in Gegenwart einer Base bzw. eines Säureacceptors durchgeführt. Man kann dazu alle üblicherweise verwendbaren anorganischen und organischen säurebindenden Mittel benützen, zu denen vorzugsweise Alkalimetall- und Erdalkalimetallcarbonate und -bicarbonate, und tertiäre Amine, z.B. Triäthylamin, Dimethylanilin und Pyridin, gehören.

Die Umsetzung nach Verfahrensvariante $a_2$) wird zweckmässigerweise in einem inerten Lösungsmittel, wie einem Aether, z.B. Diäthyläther, einem Kohlenwasserstoff, z.B. n-Hexan, oder einem chlorierten Kohlenwasserstoff, z.B. Dichlormethan oder Chloroform, und bei Raumtemperatur oder erhöhter Temperatur, z.B. bis zur Rückflusstemperatur des Reaktionsgemisches, durchgeführt. Ein bevorzugter Temperaturbereich ist derjenige zwischen 0°C und 40°C, insbesondere zwischen 10°C und 20°C.

Bei Verwendung eines Säurehalogenides der Säure der Formel II wird die Umsetzung zweckmässigerweise in Gegenwart eines säurebindenden Mittels durchgeführt. Als geeignet erweisen sich anorganische Basen, z.B. Alkalimetall- und Erdalkalimetallcarbonate und -bicarbonate, sowie organische Basen, z.B. tertiäre Amine, insbesondere Triäthylamin oder Pyridin. Das Säurehalogenid ist vorzugsweise das Säurechlorid.

Bei der Verfahrensvariante $a_3$) wird die Umsetzung zweckmässigerweise in einem inerten organischen Lösungsmittel, wie einem Aether, z.B. Tetrahydrofuran, oder einem chlorierten Kohlenwasserstoff, z.B. Dichlormethan oder Chloroform, und bei Temperaturen zwischen 0°C und 70°C durchgeführt. Ein bevorzugter Temperaturbereich ist derjenige zwischen 10°C und 30°C. Die Umsetzung erfolgt zweckmässigerweise in Gegenwart eines sauren Katalysators bzw. eines kondensierenden Mittels, wie beispielsweise Schwefelsäure, Salzsäure, p-Toluolsulfonsäure, Dicyclohexylcarbodiimid oder Carbonyldimidazol.

Die Verfahrensvariante $a_3$) eignet sich insbesondere für die Fälle, wo die Verbindung der Formel III ein Oxim ist, d.h. $R^5$ eine Gruppe (d), m, n und p die Zahl 0 und X eine Hydroxylgruppe bedeuten. Die Säure der Formel II wird mit dem Oxim der Formel III vorzugsweise in Gegenwart von Dicyclohexylcarbodiimid als kondensierendem Mittel umgesetzt. Die Säure wird zweckmässigerweise in einem inerten organischen Lösungsmittel, wie einem chlorierten Kohlenwasserstoff, z.B. Dichlormethan, Chloroform, Tetrachlorkohlenstoff oder Trichloräthan, einem Aether, z.B. Diäthyläther, Diisopropyläther oder Dioxan, oder einem aromatischen Kohlenwasserstoff, z.B. Benzol, Toluol oder Xylol, gelöst, und danach wird das Oxim in der Lösung suspendiert. Das Dicyclohexylcarbodiimid wird ebenfalls in demselben Lösungsmittel gelöst und zum Reaktionsgemisch zugegeben. Die Umsetzung erfolgt vorzugsweise in einem Temperaturbereich zwischen 0°C und der Siedetemperatur des

Reaktionsgemisches, insbesondere zwischen Raumtemperatur und 50°C. Die Reaktion ist normalerweise nach etwa 2 Stunden beendet.

Bei der Verfahrensvariante b) bedeutet der Ausdruck "Abgangsgruppe" für Y beispielsweise Chlor, Brom, Jod, Mesyloxy oder Tosyloxy. Die Umsetzung erfolgt zweckmässigerweise in einem inerten organischen Lösungsmittel, wie einem Kohlenwasserstoff, z.B. Benzol oder Toluol, einem Aether z.B. Diäthyläther, Dimethoxyäthan oder Tetrahydrofuran, oder Hexamethylphosphorsäuretriamid. Die Reaktionstemperatur ist nicht kritisch, und man arbeitet vorzugsweise bei Temperaturen zwischen -20°C und der Rückflusstemperatur des Reaktionsgemisches, insbesondere zwischen -10°C und 30°C.

In jedem Fall kann das erhaltene Produkt nach an sich bekannten Methoden isoliert und gereinigt werden.

Sofern keine gezielte Synthese zur Isolierung reiner Isomerer durchgeführt wird, fällt das Produkt normalerweise als Gemisch zweier oder mehrerer Isomerer an. Die Isomeren können nach an sich bekannten Methoden aufgetrennt werden. Gewünschtenfalls können sie auch durch Synthese aus entsprechenden optisch aktiven Ausgangsmaterialien hergestellt werden.

Die Ausgangsmaterialien der Formeln II, III, IV und V sowie reaktionsfähige Derivate der Säuren der Formel II und Alkalimetallsalze der Phenole der Formel IV sind entweder bekannt oder können in an sich bekannter Weise hergestellt werden.

Die Verbindungen der Formel I besitzen herbizide Eigenschaften und eignen sich besonders zur Bekämpfung von Ungräsern, insbesondere von Ackerfuchsschwanz (Alopecurus myosuroides) und Hirsearten, wie beispielsweise Hühnerhirse (Echinochloa crus-galli), grosse Borstenhirse (Setaria faberii) und haarartige Hirse (Panicum capillare) in Getreide, insbesondere Gerste-, Hafer- und Weizenkulturen, sowie in Reis-, Baumwolle-, Soya-, Zuckerrüben- und Gemüsekulturen. Besonders bevorzugt eignen sich die erfindungsgemässen Verbindungen zur Bekämpfung von Ungräsern in Baumwolle-, Soya-, Zuckerrüben- und Gemüsekulturen.

Im allgemeinen genügt eine Konzentration von 0,1-6 kg Wirkstoff der Formel I/ha, vorzugsweise 0,6-2 kg Wirkstoff der Formel I/ha, um den gewünschten herbiziden Effekt zu erzielen.

Die Verbindungen der Formel I sind sowohl Vorauflauf-Herbizide als auch Nachauflauf-Herbizide.

Das erfindungsgemässe Unkrautbekämpfungsmittel ist dadurch gekennzeichnet, dass es eine wirksame Menge mindestens einer Verbindung der Formel I, wie oben definiert, sowie Formulierungshilfsstoffe enthält. Das Mittel enthält zweckmässigerweise zumindest einen der folgenden Formulierungshilfsstoffe: feste Trägerstoffe; Lösungs- bzw. Dispersionsmittel; Tenside (Netz- und Emulgiermittel); Dispergatoren (ohne Tensidwirkung); und Stabilisatoren. Unter Verwendung solcher und anderer Hilfsstoffe können die Verbindungen der Formel I, also die herbiziden Wirkstoffe, in die üblichen Formulierungen übergeführt werden, wie Stäube, Pulver, Granulate, Lösungen, Emulsionen, Suspensionen, emulgierbare Konzentrate, Pasten und dergleichen.

Die Verbindungen der Formel I sind im allgemeinen wasserunlöslich und können nach den für wasserunlösliche Verbindungen üblichen Methoden unter Verwendung der diesbezüglichen Formulierungshilfsstoffe konfektioniert werden. Die Herstellung der Mittel kann in an sich bekannter Weise durchgeführt werden, z.B. durch Vermischen des jeweiligen Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln und/oder von Dispergatoren, durch Verdünnen bereits vorbereiteter emulgierbarer Konzentrate mit Lösungs- bzw. Dispersionsmitteln usw.

Als feste Trägerstoffe kommen im wesentlichen in Frage: natürliche Mineralstoffe, wie Kreide, Dolomit, Kalkstein, Tonerden und Kieselsäure und deren Salze (beispielsweise Kieselgur, Kaolin, Bentonit, Talkum, Attapulgit und Montmorillonit); synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Pulver oder als Granulate vorliegen können.

Als Lösungs- bzw. Dispersionsmittel kommen im wesentlichen in Frage: Aromaten, wie Benzol, Toluol, Xylole und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Liegt das erfindungsgemässe Unkrautbekämpfungsmittel in Form einer Druckgaspackung vor, so wird zweckmässigerweise zusätzlich zum Treibgas ein Lösungsmittel verwendet.

Die Tenside (Netz- und Emulgiermittel) können nichtionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzol-sulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäuren, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel z.B. Epichlorhydrin, Phenylglycidäther und Soyaepoxide; Antioxidantien z.B. Gallussäureester und Butylhydroxytoluol; UV-Absorber, z.B. substituierte Benzophenone, Diphenylacrylonitrilsäureester und Zimtsäureester; und Deaktivatoren, z.B. Salze der Aethylendiaminotetraessigsäure und Polyglykole.

Die erfindungsgemässen Unkrautbekämpfungsmittel können zusätzlich zu den Verbindungen der Formel I Synergisten und andere Wirkstoffe, z.B. Insektizide, Akarizide, Bakterizide, anderweitige Herbizide, Fungizide, Pflanzenwachstumsregulatoren und Düngemittel, enthalten. Solche Kombinationsmittel eignen sich zur Verstärkung der Aktivität bzw. zur Verbreiterung des Wirkungsspektrums.

Die erfindungsgemässen Unkrautbekämpfungsmittel enthalten im allgemeinen zwischen 0,1 und 99 Gewichtsprozent, vorzugsweise zwischen 5 und 75 Gewichtsprozent einer bzw. mehrerer Verbindungen der Formel I als Wirkstoff(e). Sie können z.B. in einer Form vorliegen, die sich für die Lagerung und den Transport eignet. In solchen Formulierungen, z.B. emulgierbaren Konzentraten, ist die Wirkstoffkonzentration normalerweise im höheren Bereich, vorzugsweise zwischen 10 und 75 Gewichtsprozent, insbesondere zwischen 25 und 50 Gewichtsprozent. Diese Formulierungen können dann, z.B. mit gleichen oder verschiedenen inerten Stoffen, bis zu Wirkstoffkonzentrationen verdünnt werden, die sich für den praktischen Gebrauch eignen, also vorzugsweise ca. 0,1 bis 10 Gewichtsprozent, insbesondere ca. 1 bis 5 Gewichtsprozent. Die Wirkstoffkonzentrationen können jedoch auch kleiner oder grösser sein.

Zweckmässigerweise sind in den erfindungsgemässen Unkrautbekämpfungsmitteln die festen Trägerstoffe im Konzentrationsbereich zwischen 1 und 99,9%, die Lösungs- bzw. Dispersionsmittel im Konzentrationsbereich zwischen 1 und 99,9%, die Tenside im Konzentrationsbereich zwischen 1 und

20%, die Dispergatoren im Konzentrationsbereich zwischen 1 und 20%, die Dispergatoren als Verdickungs- bzw. Antiabsetzmittel im Konzentrationsbereich zwischen 0,1 und 5% und die Stabilisatoren im Konzentrationsbereich zwischen 0,1 und 5% vorhanden, wobei diese Prozente sich auf das Gesamtgewicht des Mittels beziehen.

Wie oben erwähnt, kann die Herstellung der erfindungsgemässen Unkrautbekämpfungsmittel in an sich bekannter Weise durchgeführt werden.

Zur Herstellung pulverförmiger Präparate kann der Wirkstoff, d.h. mindestens eine Verbindung der Formel I, mit festem Trägerstoff vermischt werden, z.B. durch Zusammenmahlen; oder man kann den festen Trägerstoff mit einer Lösung oder Suspension des Wirkstoffes imprägnieren und dann die Lösungs- bzw. Dispersionsmittel durch Abdunsten, Erhitzen oder Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die Verbindung der Formel I kann auch mit einem Tensid und einem festen Trägerstoff zur Bildung eines netzbaren Pulvers vermischt werden, welches in Wasser dispergierbar ist, oder sie kann mit einem festen vorgranulierten Trägerstoff zur Bildung eines granulatförmigen Produktes vermischt werden.

Wenn gewünscht, kann die Verbindung der Formel I in einem mit Wasser nicht mischbaren Lösungsmittel, wie beispielsweise einem hochsiedenden Kohlenwasserstoff, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser

auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Unkrautbekämpfungsmittel kann nach üblichen Applikationsmethoden, wie Spritzen, Sprühen, Stäuben, Giessen oder Streuen, erfolgen. Das erfindungsgemässe Verfahren zur Bekämpfung von Unkräutern ist dadurch gekennzeichnet, dass man das gegen Unkräuter zu schützende Gut und/oder die Unkräuter mit einer erfindungsgemässen Verbindung bzw. einem erfindungsgemässen Unkrautbekämpfungsmittel behandelt.

Die nachfolgenden Beispiele dienen zur näheren Erläuterung der Erfindung.

I. Herstellung der Wirkstoffe der Formel I:

Beispiel 1

Zu einer auf 5°C abgekühlten Lösung von 1,72 g D-2-[p-(α,α,α-Trifluor-p-tolyloxy)phenoxy]-propionsäurechlorid und 0,58 g 2-Isopropylidenaminooxy-äthanol in 40 ml Benzol wird 0,47 g Pyridin zugetropft. Das Reaktionsgemisch wird während 1 Stunde bei Raumtemperatur gerührt und anschliessend unter vermindertem Druck zur Trockene eingedampft. Danach wird der Rückstand in Diäthyläther/n-Hexan (1:1) gelöst und die Lösung mit Wasser und verdünnter Natriumbicarbonat-Lösung ausgeschüttelt, über einem Trocknungsmittel getrocknet und zuletzt zur Trockene eingedampft.

Der flüssige Rückstand wird an Kieselgel mit Aceton/n-Hexan (1:7) chromatographisch gereinigt. Man erhält den D-2-[p-(α,α,α-Trifluor-p-tolyloxy)phenoxy]-propionsäure-2-isopropylidenaminooxy-äthylester; $n_D^{20}$ 1,5088; $[\alpha]_D^{22}$ +13,9° (c = 1,0% in $CHCl_3$).

In analoger Weise erhält man ausgehend von

D-2-[p-(α,α,α-Trifluor-p-tolyloxy)phenoxy]-propionsäurechlorid und N-Hydroxymethyl-acetamid den D-2-[p-(α,α,α-Trifluor-p-tolyloxy)phenoxy]-propionsäure-acetamidomethylester; Fp. 103-105°C; $[\alpha]_D^{22}$ +13,5° (c = 1,1% in $CHCl_3$).

D-2-[p-(α,α,α-Trifluor-p-tolyloxy)phenoxy]-propionsäurechlorid und N-Hydroxymethyl-chloracetamid den D-2-[p-(α,α,α-Trifluor-p-tolyloxy)phenoxy]-propionsäure-chloracetamidomethylester; Fp. 98-99°C; $[\alpha]_D^{22}$ +19,8° (c = 0,7% in $CHCl_3$).

D-2-[p-(α,α,α-Trifluor-p-tolyloxy)phenoxy]-propionsäurechlorid und N-Hydroxymethyl-trichloracetamid den D-2-[p-(α,α,α-Trifluor-p-tolyloxy)phenoxy]-propionsäure-tri-

chloracetamidomethylester; $n_D^{20}$ 1,5320; $[\alpha]_D^{20}$ +14,8° (c = 0,6% in $CHCl_3$).

D-2-[p-(α,α,α-Trifluor-p-tolyloxy)phenoxy]-propionsäurechlorid und 2-Methansulfonamido-äthanol den D-2-[p-(α,α,α-Trifluor-p-tolyloxy)phenoxy]-propionsäure-2-methansulfonamido-äthylester; Fp. 65-68°C; $[\alpha]_D^{22}$ +24,6° (c = 1,1% in $CHCl_3$).

D-2-[p-(α,α,α-Trifluor-p-tolyloxy)phenoxy]-propionsäurechlorid und N-Hydroxymethyl-succinimid den D-2-[p-(α,α,α-Trifluor-p-tolyloxy)phenoxy]-propionsäure-succinimidomethylester; Fp. 74-76°C; $[\alpha]_D^{22}$ +13,5° (c = 1,3% in $CHCl_3$).

D-2-[p-(α,α,α-Trifluor-p-tolyloxy)phenoxy]-propionsäurechlorid und N-Hydroxymethyl-phthalimid den D-2-[p-(α,α,α-Trifluor-p-tolyloxy)phenoxy]-propionsäure-phthalimidomethylester; Fp. 99-100°C; $[\alpha]_D^{22}$ -4,9° (c = 0,7% in $CHCl_3$).

D-2-[p-(α,α,α-Trifluor-p-tolyloxy)phenoxy]-propionsäurechlorid und Diäthoxyphosphinylmethanol den D-2-[p-(α,α,α-Trifluor-p-tolyloxy)phenoxy]-propionsäure-diäthoxyphosphinylmethylester; $n_D^{20}$ 1,5008; $[\alpha]_D^{22}$ +27,5° (c = 1,0% in $CHCl_3$).

D-2-[p-(α,α,α-Trifluor-p-tolyloxy)phenoxy]-propionsäurechlorid und 2-Diäthoxyphosphinyl-äthanol den D-2-[p-(α,α,α-Trifluor-p-tolyloxy)phenoxy]-propionsäure-2-diäthoxyphosphinyl-äthylester; $n_D^{20}$ 1,5001; $[\alpha]_D^{22}$ +20,7° (c = 0,7% in $CHCl_3$).

D-2-[p-(α,α,α-Trifluor-p-tolyloxy)phenoxy]-propionsäurechlorid und 1-Diäthoxyphosphinyl-äthanol den D-2-[p-(α,α,α-Trifluor-p-tolyloxy)phenoxy]-propionsäure-1-diäthoxyphosphinyl-äthylester; $n_D^{20}$ 1,4963; $[\alpha]_D^{22}$ +20,3° (c = 1,3% in $CHCl_3$).

D-2-[p-(α,α,α-Trifluor-p-tolyloxy)phenoxy]-propionsäurechlorid und 1-Diäthoxyphosphinyl-n-butanol den D-2-[p-(α,α,α-Trifluor-p-tolyloxy)phenoxy]-propionsäure-1-diäthoxyphosphinyl-n-butylester; $n_D^{20}$ 1,4945; $[\alpha]_D^{22}$ +23,0° (c = 1,4% in $CHCl_3$).

D-2-[p-(α,α,α-Trifluor-p-tolyloxy)phenoxy]-propionsäurechlorid und α-Diäthoxyphosphinyl-benzylalkohol den D-2-[p-(α,α,α-Trifluor-p-tolyloxy)phenoxy]-propionsäure-α-diäthoxyphosphinyl-benzylester; $n_D^{20}$ 1,5228; $[\alpha]_D^{22}$ +25,5° (c = 0,7% in $CHCl_3$).

D-2-[p-(p-Jodphenoxy)phenoxy]-propionsäurechlorid und 2-Isopropylidenaminooxy-äthanol den D-2-[p-(p-Jodphenoxy)phenoxy]-propionsäure-2-isopropylidenaminooxy-äthylester; $[\alpha]_D^{22}$+12,6° (c = 1,2% in $CHCl_3$).

D-2-[p-(p-Jodphenoxy)phenoxy]-propionsäurechlorid und 2-Methansulfonamido-äthanol den D-2-[p-(p-Jodphenoxy)-phenoxy]-propionsäure-2-methansulfonamido-äthylester; Fp. 84-87°C; $[\alpha]_D^{22}$ +23,2° (c = 1,0% in $CHCl_3$).

D-2-[p-(p-Jodphenoxy)phenoxy]-propionsäurechlorid und N-Hydroxymethyl-succinimid den D-2-[p-(p-Jodphenoxy)-phenoxy]-propionsäure-succinimidomethylester; $[\alpha]_D^{22}$+11,5° (c = 0,8% in $CHCl_3$).

D-2-[p-(p-Jodphenoxy)phenoxy]-propionsäurechlorid und Diäthoxyphosphinylmethanol den D-2-[p-(p-Jodphenoxy)-phenoxy]-propionsäure-diäthoxyphosphinylmethylester; $[\alpha]_D^{22}$ + 23,7° (c = 1,7% in $CHCl_3$).

D-2-[p-(p-Jodphenoxy)phenoxy]-propionsäurechlorid und 2-Diäthoxyphosphinyl-äthanol den D-2-[p-(p-Jodphenoxy)-phenoxy]-propionsäure-2-diäthoxyphosphinyl-äthylester; $[\alpha]_D^{22}$ +19,7° (c = 1,3% in $CHCl_3$).

D-2-[p-(o-Chlor-p-jodphenoxy)phenoxy]-propionsäure-chlorid und 2-Isopropylidenaminooxy-äthanol den D-2-[p-(o-Chlor-p-jodphenoxy)phenoxy]-propionsäure-2-isopropylidenaminooxy-äthylester; $[\alpha]_D^{20}$ + 11,7° (c = 0,9% in $CHCl_3$).

D-2-[p-(o-Chlor-p-jodphenoxy)phenoxy]-propionsäure-chlorid und 2-Methansulfonamido-äthanol den D-2-[p-(o-Chlor-p-jodphenoxy)phenoxy]-propionsäure-2-methansulfon-amido-äthylester; $[\alpha]_D^{20}$ + 19,4° (c = 0,8% in $CHCl_3$).

D-2-[p-(o-Chlor-p-jodphenoxy)phenoxy]-propionsäure-chlorid und Diäthoxyphosphinylmethanol den D-2-[p-(o-Chlor-p-jodphenoxy)phenoxy]-propionsäure-diäthoxyphosphinyl-methylester; $[\alpha]_D^{20}$ + 19,7° (c = 1,6% in $CHCl_3$).

D-2-[p-(α,α,α-Trifluor-p-tolyloxy)phenoxy]-propion-säurechlorid und Dibutoxyphosphinylmethanol den D-2-[p-(α,α,α-Trifluor-p-tolyloxy)phenoxy]-propionsäure-dibutoxy-phosphinylmethylester; $n_D^{20}$ 1,4932; $[\alpha]_D^{20}$ +23,2° (c = 0,60% in $CHCl_3$).

D-2-[p-(α,α,α-Trifluor-p-tolyloxy)phenoxy]-propion-säurechlorid und 1-Diphenoxyphosphinyl-äthanol den D-2-[p-(α,α,α-Trifluor-p-tolyloxy)phenoxy]-propionsäure-1-diphenoxyphosphinyl-äthylester; $n_D^{20}$ 1,5449; $[\alpha]_D^{22}$ +7,3° (c = 1,1% in $CHCl_3$).

D-2-[p-(α,α,α-Trifluor-p-tolyloxy)phenoxy]-propionsäurechlorid und 1-Isopropylidenaminooxy-2-propanol den D-2-[p-(α,α,α-Trifluor-p-tolyloxy)phenoxy]-propionsäure-1-isopropylidenaminooxy-2-propylester; $n_D^{20}$ 1,5026; $[\alpha]_D^{22}$ +19,0° (c = 1,1% in $CHCl_3$).

D-2-[p-(α,α,α-Trifluor-p-tolyloxy)phenoxy]-propionsäurechlorid und 1-Isopropylidenaminooxy-2-butanol den D-2-[p-(α,α,α-Trifluor-p-tolyloxy)phenoxy]-propionsäure-1-isopropylidenaminooxy-2-butylester; $n_D^{20}$ 1,5023; $[\alpha]_D^{22}$ +20,7° ( c = 1,0% in $CHCl_3$).

D-2-[p-(α,α,α-Trifluor-p-tolyloxy)phenoxy]-propionsäurechlorid und 2-Isopropylidenaminooxy-1-phenyl-äthanol den D-2-[p-(α,α,α-Trifluor-p-tolyloxy)phenoxy]-propionsäure-2-isopropylidenaminooxy-1-phenyläthylester; $[\alpha]_D^{22}$ +19,8° (c = 0,9% in $CHCl_3$).

D-2-[p-(α,α,α-Trifluor-p-tolyloxy)phenoxy]-propionsäurechlorid und 2-(2-Butylidenaminooxy)-äthanol den D-2-[p-(α,α,α-Trifluor-p-tolyloxy)phenoxy]-propionsäure-2-(2-butylidenaminooxy)-äthylester; $n_D^{20}$ 1,5072; $[\alpha]_D^{22}$ +13,7° (c = 1,1% in $CHCl_3$).

D-2-[p-(α,α,α-Trifluor-p-tolyloxy)phenoxy]-propionsäurechlorid und 2-(α-Methylbenzylidenaminooxy)-äthanol den D-2-[p-(α,α,α-Trifluor-p-tolyloxy)phenoxy]-propionsäure-2-(α-methylbenzylidenaminooxy)-äthylester; $n_D^{20}$ 1,5456; $[\alpha]_D^{22}$ +7,6° (c = 1,0% in $CHCl_3$).

D-2-[p-(α,α,α-Trifluor-p-tolyloxy)phenoxy]-propionsäurechlorid und 2-Cyclohexylidenaminooxy-äthanol den D-2-[p-(α,α,α-Trifluor-p-tolyloxy)phenoxy]-propionsäure-2-cyclohexylidenaminooxy-äthylester; $n_D^{20}$ 1,5201; $[\alpha]_D^{20}$ +11,7° (c = 1,1% in $CHCl_3$).

Beispiel 2

3,0 g D-2-[p-(α,α,α-Trifluor-p-tolyloxy)phenoxy]-propionsäure werden in 10 ml Dichlormethan suspendiert, und bei Raumtemperatur werden 1,3 g Acetessigsäure-äthylester-oxim zugegeben. Zu der Suspension wird während 10 Minuten eine Lösung von 1,9 g Dicyclohexylcarbodiimid in 10 ml Dichlormethan zugetropft. Dabei steigt die Temperatur des Reaktionsgemisches auf 40°C. Das Gemisch wird 2 Stunden bei Raumtemperatur nachgerührt und anschliessend filtriert und unter vermindertem Druck zur Trockene eingedampft. Man erhält 3-/D-2-[p-(α,α,α-Trifluor-p-tolyloxy)phenoxy]-propionoxyimino/-buttersäure-äthylester; $[\alpha]_D^{20}$ +39,8° (c = 1,0% in $CHCl_3$).

In analoger Weise erhält man ausgehend von

D-2-[p-(α,α,α-Trifluor-p-tolyloxy)phenoxy]-propionsäure und 1,1,1-Trifluoracetonoxim den D-2-[p-(α,α,α-Trifluor-p-tolyloxy)phenoxy]-propionsäure-1,1,1-trifluoracetonoximester; $[\alpha]_D^{20}$ +39,5° (c = 1,0% in $CHCl_3$).

D-2-[p-(α,α,α-Trifluor-p-tolyloxy)phenoxy]-propionsäure und 1-Hydroxyimino-diäthyläther den Aethyl-N-/D-2-[p-(α,α,α-Trifluor-p-tolyloxy)phenoxy]-propionoxy/-acetimidat; $n_D^{20}$ 1,5087; $[\alpha]_D^{22}$ +53° (c = 0,9% in $CHCl_3$).

D-2-[p-(α,α,α-Trifluor-p-tolyloxy)phenoxy]-propionsäure und n-Butyl-(1-hydroxyiminoäthyl)-äther den n-Butyl-N-/D-2-[p-(α,α,α-trifluor-p-tolyloxy)phenoxy]-propionoxy/-acetimidat; $n_D^{20}$ 1,5036; $[\alpha]_D^{22}$ +49,87° (c = 1,2% in $CHCl_3$).

D-2-[p-(α,α,α-Trifluor-p-tolyloxy)phenoxy]-propionsäure und Aethyl-(1-hydroxyimino-n-butyl)-äther den Aethyl-N-/D-2-[p-(α,α,α-trifluor-p-tolyloxy)phenoxy]-propionoxy/-n-butyrimidat; $n_D^{20}$ 1,5058; $[\alpha]_D^{20}$ +52,58° (c = 1,0% in $CHCl_3$).

D-2-[p-(α,α,α-Trifluor-p-tolyloxy)phenoxy]-propionsäure

und Methoxyacetonoxim den D-2-[p-(α,α,α-Trifluor-p-tolyloxy)-phenoxy]-propionsäure-methoxyacetonoximester; $[\alpha]_D^{20}$ +48,77° (c = 1,9% in $CHCl_3$).

D-2-[p-(α,α,α-Trifluor-p-tolyloxy)phenoxy]-propionsäure und Diacetonmonoxim den D-2-[p-(α,α,α-Trifluor-p-tolyloxy)-phenoxy]-propionsäure-diacetonmonoximester; $[\alpha]_D^{20}$ +30,7° (c = 1,0% in $CHCl_3$).

D-2-[p-(α,α,α-Trifluor-p-tolyloxy)phenoxy]-propionsäure und Brenztraubensäure-äthylester-oxim den 2-/D-2-[p-(α,α,α-Trifluor-p-tolyloxy)phenoxy]-propionoxyimino/-propionsäure-äthylester; $[\alpha]_D^{20}$ +45,01° (c = 2,0% in $CHCl_3$).

D-2-[p-(α,α,α-Trifluor-p-tolyloxy)phenoxy-propionsäure und α-Methyl-acetessigsäure-äthylester-oxim den 2-Methyl-3-/D-2-[p-(α,α,α-trifluor-p-tolyloxy)phenoxy]-propionoxy-imino/-buttersäure-äthylester; $[\alpha]_D^{20}$ +41,07° (c = 0,9% in $CHCl_3$).

D-2-[p-(α,α,α-Trifluor-p-tolyloxy)phenoxy]-propionsäure und Lävulinsäure-äthylester-oxim den 4-/D-2-[p-(α,α,α-Trifluor-p-tolyloxy)phenoxy]-propionoxyimino/-n-valeriansäure-äthylester; $[\alpha]_D^{20}$ +37,4° (c = 1,2% in $CHCl_3$).

D-2-[p-(o-Chlor-p-trifluormethyl-phenoxy)phenoxy]-propionsäure und 2-Isopropylidenaminooxy-äthanol den D-2-[p-(o-Chlor-p-trifluormethyl-phenoxy)phenoxy]-propionsäure-2-isopropylidenaminooxy-äthylester; $[\alpha]_D^{20}$ +12,3° (c = 0,69% in $CHCl_3$).

D-2-[p-(α,α,α-Trifluor-p-tolyloxy)phenoxy]-propionsäure und Cyclopentanonoxim den D-2-[p-(α,α,α-Trifluor-p-tolyloxy)phenoxy]-propionsäure-cyclopentanonoximester; $[\alpha]_D^{20}$ +40,8° (c = 1,0% in $CHCl_3$).

Beispiel 3

Eine Lösung von 1,79 g D-2-[p-(α,α,α-Trifluor-p-tolyloxy)phenoxy]-propionsäure, 1,30 g 1-Diäthoxyphosphinyl-cyclohexanol und 0,06 g 4-Dimethylamino-pyridin in 20 ml Dichlormethan wird bei Raumtemperatur mit 1,13 g Dicyclohexylcarbodiimid versetzt und 24 Stunden gerührt. Das Reaktionsgemisch wird unter vermindertem Druck zur Trockene eingedampft, der Rückstand in Diäthyläther/n-Hexan (1:1) gelöst und der unlösliche Teil des Rückstandes abgenutscht. Anschliessend wird das Filtrat zweimal mit je 30 ml 5%iger Essigsäure und dreimal mit je 30 ml Wasser gewaschen, über einem Trocknungsmittel getrocknet und zur Trockene eingedampft. Der flüssige Rückstand wird an Kieselgel mit Dichlormethan/Diäthyläther (1:1) chromatographisch gereinigt. Man erhält den D-2-[p-(α,α,α-Trifluor-p-tolyloxy)phenoxy]-propionsäure-1-diäthoxyphosphinyl-cyclohexylester; $n_D^{20}$ 1,5073.

In analoger Weise erhält man ausgehend von

D-2-[p-(α,α,α-Trifluor-p-tolyloxy)phenoxy]-propionsäure und 2-Dimethoxyphosphinyl-2-propanol den D-2-[p-(α,α,α-Trifluor-p-tolyloxy)phenoxy]-propionsäure-2-dimethoxyphosphinyl-2-propylester; $n_D^{20}$ 1,5019; $[\alpha]_D^{22}$ +7,6° (c = 1,2% in $CHCl_3$).

Beispiel 4

Ein Gemisch aus 1,89 g D-2-[p-(α,α,α-Trifluor-p-tolyloxy)phenoxy]-propionsäure, 1,43 g 3-Diäthoxyphosphinylpropylbromid, 0,40 g Kaliumcarbonat und 50 ml absolutem Aceton wird 15 Stunden auf Rückflusstemperatur erhitzt. Danach wird das Reaktionsgemisch unter vermindertem Druck zur Trockene eingedampft und der Rückstand in Diäthyläther gelöst. Die Lösung wird zweimal mit je 25 ml Wasser gewaschen und zweimal mit je 25 ml Natriumbicarbonat-Lösung ausgeschüttelt, über einem Trocknungsmittel getrocknet und

zur Trockene eingedampft.

Der flüssige Rückstand wird an Kieselgel mit Dichlormethan chromatographisch gereinigt. Man erhält D-2-[p-(α,α,α-Trifluor-p-tolyloxy)phenoxy]-propionsäure-3-diäthoxyphosphinyl-propylester; $n_D^{20}$ 1,4986; $[\alpha]_D^{22}$ +20,52° (c = 0,9% in $CHCl_3$).

In analoger Weise erhält man ausgehend von

D-2-[p-(p-Jodphenoxy)phenoxy]-propionsäure und 3-Diäthoxyphosphinylpropylbromid den D-2-[p-(p-Jodphenoxy)-phenoxy]-propionsäure-3-diäthoxyphosphinyl-propylester; $[\alpha]_D^{22}$ +20,4° (c = 1,2% in $CHCl_3$).

II. Formulierungsbeispiele:

Beispiel 5

Zur Herstellung eines emulgierbaren Konzentrates werden die nachstehend aufgeführten Bestandteile miteinander vermischt:

| | |
|---|---|
| Verbindung der Formel I | 500 g |
| Gemisch aus Kondensationsprodukten eines Alkylphenols und Aethylenoxid und Dodecylbenzolsulfon-saurem Calcium | 100 g |
| Epoxydiertes Soyaöl mit einem Oxiransauerstoffgehalt von ca. 6% | 25 g |
| Butyliertes Hydroxytoluol | 10 g |

Diese Mischung wird mit Xylol auf 1 Liter aufgefüllt.

Das so erhaltene Konzentrat emulgiert spontan in Wasser. Die sich ergebende Emulsion eignet sich als gebrauchsfertige Spritzbrühe.

## Beispiel 6

Zur Herstellung eines emulgierbaren Konzentrates werden die nachstehend aufgeführten Bestandteile miteinander vermischt:

| | |
|---|---|
| Verbindung der Formel I | 250 g/l |
| N-Methyl-2-pyrrolidon | 300 g/l |
| Emulgator A[1)] | 100 g/l |
| Emulgator B[2)] | 25 g/l |
| Lösungsmittelgemisch aus Alkylbenzolen | auf 1000 ml |

[1)]Emulgator A: Emulgator bestehend aus 60 Teilen eines Blockpolymerisates aus Aethylenoxid und Propylenoxid, 20 Teilen des Calciumsalzes einer verzweigtkettigen Dodecylbenzolsulfonsäure und 20 Teilen eines Lösungsmittelgemisches aus Isobutanol und $C_{10}$-Alkylbenzolen.

[2)]Emulgator B: Gemisch aus 70 Teilen des Calciumsalzes einer verzweigtkettigen Dodecylbenzolsulfonsäure und 30 Teilen eines Lösungsmittelgemisches aus Isobutanol und $C_{10}$-Alkylbenzolen.

Das so erhaltene Konzentrat emulgiert spontan in Wasser. Die sich ergebende Emulsion eignet sich als gebrauchsfertige Spritzbrühe.

## III. Biologische Befunde:

## Beispiel 7

Die Formulierung des Beispiels 6 wird unter stetigem Verdünnen mit Wasser, das 0,5 Volumenprozent Nonoxynol (Kondensationsprodukt aus Nonylphenol und Aethylenoxid) enthält, auf die Versuchsunkräuter (Lolium multiflorum bzw. Bromus inermis) gesprüht. Das Besprühen erfolgt mittels eines logarithmischen Sprühgeräts, das ermöglicht, die Konzentration des Wirkstoffes nach bestimmten Abständen

automatisch zu halbieren. Das Sprühvolumen beträgt 500 l/ha. 33 Tage nach dem Besprühen werden die Unkräuter auf die eingetretene Wirkung untersucht, d.h. es wird die Dosierung bestimmt, bei der eine ausreichende Kontrolle der Unkräuter erzielt wird. Die Ergebnisse sind in der nachstehenden Tabelle I zusammengefasst.

Tabelle I

| Wirkstoff der Formel I | Dosierung (g Wirkstoff/ha) | |
|---|---|---|
| | Lolium multiflorum | Bromus inermis |
| Aethyl-N-/D-2-[p-(α,α,α-trifluor-p-tolyloxy)-phenoxy]-propionoxy/-n-butyrimidat | 165 | 165 |
| n-Butyl-N-/D-2-[p-(α,α,α-trifluor-p-tolyloxy)-phenoxy]-propionoxy/-acetimidat | 125 | 220 |

Beispiel 8

Der Wirkstoff wird in der Formulierung des Beispiels 6 geprüft. Kurz vor Versuchsbeginn wird mit Wasser, das 0,5 Volumenprozent Nonoxynol als Netzmittel enthält, auf die gewünschte Konzentration verdünnt.

Die Testunkräuter (Oryza sativa) im 2 Blattstadium werden im Gewächshaus mit dem so formulierten Wirkstoff besprüht. Während der Nachperiode wird mittels Quecksilberdampflampen jeweils ein 16-Stunden-Tag simuliert. 3 Wochen nach dem Besprühen werden die Unkräuter auf die eingetretene Wirkung untersucht, d.h. es wird die prozentuale Nekrose bestimmt, wobei 100%ige Nekrose einer vollständigen Zerstörung der Unkräuter entspricht. Die Ergebnisse sind in der nachstehenden Tabelle II zusammengefasst.

Tabelle II

| Wirkstoff der Formel I | Dosierung g/ha | %ige Nekrose (Oryza sativa) |
|---|---|---|
| D-2-[p-(α,α,α-Trifluor-p-tolyloxy)phenoxy]-propionsäure-methoxy-acetonoximester | 150 | 90 |

## Patentansprüche

1. Verbindungen der allgemeinen Formel

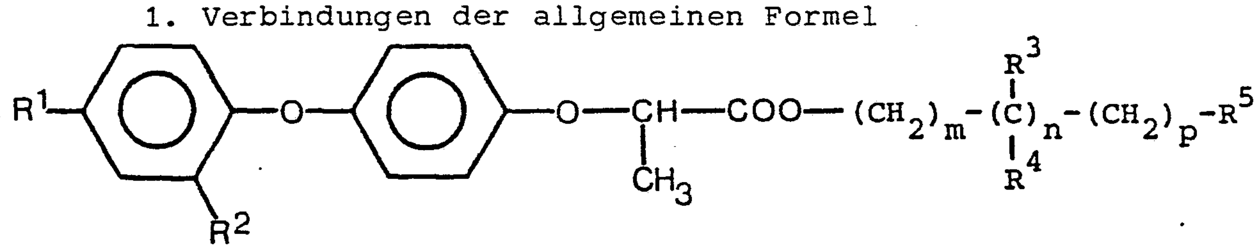

I

worin $R^1$ Chlor, Brom, Jod oder Trifluormethyl,
$R^2$ Wasserstoff, Chlor, Brom oder Jod,
$R^3$ Wasserstoff oder $C_{1-4}$-Alkyl,
$R^4$ Wasserstoff; $C_{1-4}$-Alkyl; oder Phenyl, gegebenenfalls substituiert mit Halogen, $C_{1-4}$-Alkyl und/oder Nitro,
wobei $R^3$ und $R^4$ zusammen mit dem Kohlenstoffatom, an das sie geknüpft sind, auch einen $C_{5-8}$-Cycloalkanring bilden können, der gegebenenfalls mit 1 bis 3 $C_{1-4}$-Alkylgruppen substituiert sein kann,
m, n und p die Zahl 0 oder 1
und $R^5$ eine der folgenden Gruppen (a) - (f) bedeuten:

$-NHCO-R^6$ (a)

$-NHSO_2-R^7$ (b)

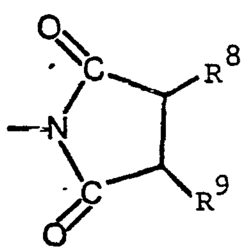

(c)

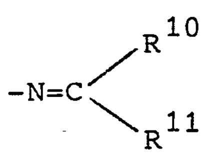

(d)

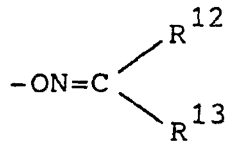

(e)

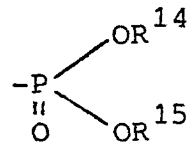

(f)

wobei in den obigen Formeln der Gruppen (a) - (f)

$R^6$ Wasserstoff; $C_{1-6}$-Alkyl; $C_{1-4}$-Halogenalkyl; oder Phenyl, gegebenenfalls substituiert mit Halogen, $C_{1-4}$-Alkyl und/oder Nitro bedeutet,

$R^7$ $C_{1-6}$-Alkyl; oder Phenyl, gegebenenfalls substituiert mit Halogen, $C_{1-4}$-Alkyl und/oder Nitro bedeutet,

$R^8$ und $R^9$ für Wasserstoff stehen oder zusammen eine Bindung, Trimethylen, Tetramethylen, 2-Butenylen oder einen ankondensierten Benzolring bedeuten,

$R^{10}$ $C_{1-6}$-Alkyl und $R^{11}$ Trifluormethyl, $C_{1-6}$-Alkoxy, $C_{1-6}$-Alkoxy-$C_{1-4}$-alkyl, $C_{2-7}$-Alkanoyl, $C_{2-7}$-Alkoxycarbonyl oder $C_{2-7}$-Alkoxycarbonyl-$C_{1-4}$-alkyl bedeuten, oder $R^{10}$ und $R^{11}$ zusammen mit dem Kohlenstoffatom, an das sie geknüpft sind, einen Cyclopentanring bilden, der gegebenenfalls mit 1 bis 3 $C_{1-4}$-Alkyl und/oder 1 $C_{1-4}$-Alkoxy substituiert sein kann, wobei, falls $R^{10}$, $R^{11}$ und das Kohlenstoffatom einen gegebenenfalls substituierten Cyclopentanring bilden, $R^1$ Chlor, Brom oder Trifluormethyl ist,

$R^{12}$ Wasserstoff oder $C_{1-6}$-Alkyl und $R^{13}$ $C_{1-6}$-Alkyl; oder Phenyl, gegebenenfalls substituiert mit Halogen, $C_{1-4}$-Alkyl und/oder Nitro bedeuten, oder $R^{12}$ und $R^{13}$ zusammen mit dem Kohlenstoffatom, an das sie geknüpft sind, einen $C_{5-7}$-Cycloalkanring bilden, und

$R^{14}$ und $R^{15}$ $C_{1-6}$-Alkyl oder Phenyl bedeuten,

und wobei, wenn $R^5$ eine Gruppe (a), (b) oder (c) darstellt, m die Zahl 1, n die Zahl 0 und p die Zahl 0 oder 1 bedeuten; wenn $R^5$ eine Gruppe (d) darstellt,

m, n und p die Zahl 0 bedeuten; wenn $R^5$ eine Gruppe (e) darstellt, m die Zahl 0 und n und p die Zahl 1 bedeuten; und wenn $R^5$ eine Gruppe (f) darstellt, m und p die Zahl 0 oder 1 und n die Zahl 1 bedeuten.

2. Verbindungen nach Anspruch 1, worin $R^1$ Jod oder Trifluormethyl bedeutet.

3. Verbindungen nach Anspruch 1 oder 2, worin $R^2$ Wasserstoff oder Chlor bedeutet.

4. Die D-Isomeren der Verbindungen nach einem der Ansprüche 1 bis 3.

5. D-2-[p-(α,α,α-Trifluor-p-tolyloxy)phenoxy]-propionsäure-succinimidomethylester.

6. Aethyl-N-/D-2-[p-(α,α,α-trifluor-p-tolyloxy)-phenoxy]-propionoxy/-acetimidat.

7. n-Butyl-N-/D-2-[p-(α,α,α-trifluor-p-tolyloxy)-phenoxy]-propionoxy/-acetimidat.

8. D-2-[p-(α,α,α-Trifluor-p-tolyloxy)phenoxy]-propionsäure-2-isopropylidenaminooxy-äthylester.

9. D-2-[p-(α,α,α-Trifluor-p-tolyloxy)phenoxy]-propionsäure-diäthoxyphosphinylmethylester.

10. Eine Verbindung nach Anspruch 1, ausgewählt unter:

D-2-[p-(α,α,α-Trifluor-p-tolyloxy)phenoxy]-propionsäure-acetamidomethylester,

D-2-[p-(α,α,α-Trifluor-p-tolyloxy)phenoxy]-propionsäure-chloracetamidomethylester,

D-2-[p-(α,α,α-Trifluor-p-tolyloxy)phenoxy]-propionsäure-trichloracetamidomethylester,

D-2-[p-(α,α,α-Trifluor-p-tolyloxy)phenoxy]-propionsäure-3-diäthoxyphosphinyl-propylester.

11. Eine Verbindung nach Anspruch 1, ausgewählt unter:

D-2-[p-(p-Jodphenoxy)phenoxy]-propionsäure-2-isopropylidenaminooxy-äthylester,
D-2-[p-(p-Jodphenoxy)phenoxy]-propionsäure-2-methansulfonamido-äthylester,
D-2-[p-(p-Jodphenoxy)phenoxy]-propionsäure-succinimidomethylester,
D-2-[p-(p-Jodphenoxy)phenoxy]-propionsäure-diäthoxyphosphinylmethylester,
D-2-[p-(p-Jodphenoxy)phenoxy]-propionsäure-2-diäthoxyphosphinyl-äthylester,
D-2-[p-(o-Chlor-p-jodphenoxy)phenoxy]-propionsäure-2-isopropylidenaminooxy-äthylester,
D-2-[p-(o-Chlor-p-jodphenoxy)phenoxy]-propionsäure-2-methansulfonamido-äthylester,
D-2-[p-(o-Chlor-p-jodphenoxy)phenoxy]-propionsäure-diäthoxyphosphinylmethylester,

D-2-[p-(α,α,α-Trifluor-p-tolyloxy)phenoxy]-propionsäure-dibutoxyphosphinylmethylester,
D-2-[p-(α,α,α-Trifluor-p-tolyloxy)phenoxy]-propionsäure-1-diphenoxyphosphinyl-äthylester,
D-2-[p-(α,α,α-Trifluor-p-tolyloxy)phenoxy]-propionsäure-1-isopropylidenaminooxy-2-propylester,
D-2-[p-(α,α,α-Trifluor-p-tolyloxy)phenoxy]-propionsäure-1-isopropylidenaminooxy-2-butylester,
D-2-[p-(α,α,α-Trifluor-p-tolyloxy)phenoxy]-propionsäure-1-isopropylidenaminooxy-1-phenyläthylester,
D-2-[p-(α,α,α-Trifluor-p-tolyloxy)phenoxy]-propionsäure-2-(2-butylidenaminooxy)-äthylester,
D-2-[p-(α,α,α-Trifluor-p-tolyloxy)phenoxy]-propionsäure-2-(α-methylbenzylidenaminooxy)-äthylester,
D-2-[p-(α,α,α-Trifluor-p-tolyloxy)phenoxy]-propion-

D-2-[p-(α,α,α-Trifluor-p-tolyloxy)phenoxy]-propionsäure-2-methansulfonamido-äthylester,

D-2-[p-(α,α,α-Trifluor-p-tolyloxy)phenoxy]-propionsäure-phthalimidomethylester,

D-2-[p-(α,α,α-Trifluor-p-tolyloxy)phenoxy]-propionsäure-2-diäthoxyphosphinyl-äthylester,

D-2-[p-(α,α,α-Trifluor-p-tolyloxy)phenoxy]-propionsäure-1-diäthoxyphosphinyl-äthylester,

D-2-[p-(α,α,α-Trifluor-p-tolyloxy)phenoxy]-propionsäure-1-diäthoxyphosphinyl-n-butylester,

D-2-[p-(α,α,α-Trifluor-p-tolyloxy)phenoxy]-propionsäure-α-diäthoxyphosphinyl-benzylester,

3-/D-2-[p-(α,α,α-Trifluor-p-tolyloxy)phenoxy]-propionoxyimino/-buttersäure-äthylester,

D-2-[p-(α,α,α-Trifluor-p-tolyloxy)phenoxy]-propionsäure-1,1,1-trifluoracetonoximester,

Aethyl-N-/D-2-[p-(α,α,α-trifluor-p-tolyloxy)phenoxy]-propionoxy/-n-butyrimidat,

D-2-[p-(α,α,α-Trifluor-p-tolyloxy)phenoxy]-propionsäure-methoxyacetonoximester,

D-2-[p-(α,α,α-Trifluor-p-tolyloxy)phenoxy]-propionsäure-diacetonmonoximester,

2-/D-2-[p-(α,α,α-Trifluor-p-tolyloxy)phenoxy]-propionoxyimino/-propionsäure-äthylester,

2-Methyl-3-/D-2-[p-(α,α,α-trifluor-p-tolyloxy)-phenoxy]-propionoxyimino/-buttersäure-äthylester,

4-/D-2-[p-(α,α,α-Trifluor-p-tolyloxy)phenoxy]-propionoxyimino/-n-valeriansäure-äthylester,

D-2-[p-(o-Chlor-p-trifluormethyl-phenoxy)phenoxy]-propionsäure-2-isopropylidenaminooxy-äthylester,

D-2-[p-(α,α,α-Trifluor-p-tolyloxy)phenoxy]-propionsäure-cyclopentanonoximester,

D-2-[p-(α,α,α-Trifluor-p-tolyloxy)phenoxy]-propionsäure-1-diäthoxyphosphinyl-cyclohexylester,

D-2-[p-(α,α,α-Trifluor-p-tolyloxy)phenoxy]-propionsäure-2-dimethoxyphosphinyl-2-propylester und

säure-2-cyclohexylidenaminooxy-äthylester und

D-2-[p-(p-Jodphenoxy)phenoxy]-propionsäure-3-diäthoxy-phosphinyl-propylester.

12. Eine Verbindung nach einem der Ansprüche 1 bis 10 als Unkrautbekämpfungsmittel.

13. Eine Verbindung nach Anspruch 11 als Unkrautbekämpfungsmittel.

14. Unkrautbekämpfungsmittel, dadurch gekennzeichnet, dass es eine wirksame Menge mindestens einer Verbindung der allgemeinen Formel

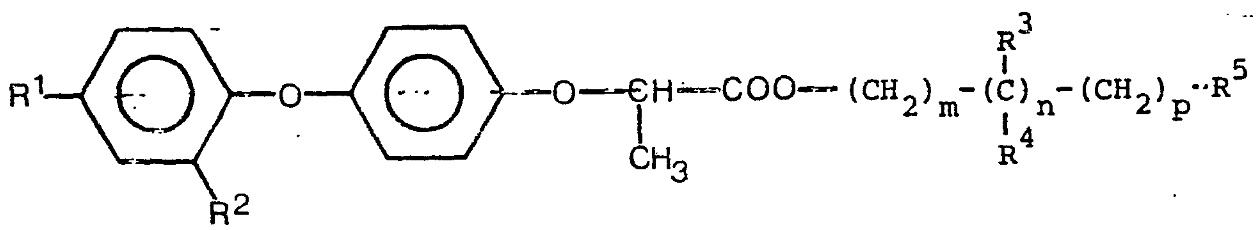

I

worin $R^1$ Chlor, Brom, Jod oder Trifluormethyl,
$R^2$ Wasserstoff, Chlor, Brom oder Jod,
$R^3$ Wasserstoff oder $C_{1-4}$-Alkyl,
$R^4$ Wasserstoff; $C_{1-4}$-Alkyl; oder Phenyl, gegebenenfalls substituiert mit Halogen, $C_{1-4}$-Alkyl und/oder Nitro,
wobei $R^3$ und $R^4$ zusammen mit dem Kohlenstoffatom, an das sie geknüpft sind, auch einen $C_{5-8}$-Cycloalkanring bilden können, der gegebenenfalls mit 1 bis 3 $C_{1-4}$-Alkylgruppen substituiert sein kann,
m, n und p die Zahl 0 oder 1
und $R^5$ eine der folgenden Gruppen (a) - (f) bedeuten:

$-NHCO-R^6$ (a)

$-NHSO_2-R^7$ (b)

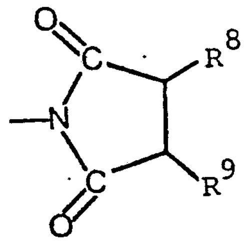

(c)

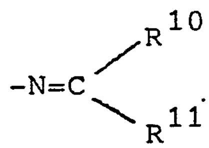

(d)

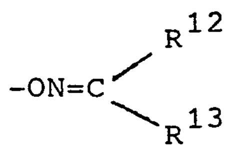

(e)

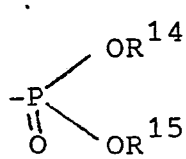

(f)

wobei in den obigen Formeln der Gruppen (a) - (f)
$R^6$ Wasserstoff; $C_{1-6}$-Alkyl; $C_{1-4}$-Halogenalkyl; oder Phenyl, gegebenenfalls substituiert mit Halogen, $C_{1-4}$-Alkyl und/oder Nitro bedeutet,
$R^7$ $C_{1-6}$-Alkyl; oder Phenyl, gegebenenfalls substituiert mit Halogen, $C_{1-4}$-Alkyl und/oder Nitro bedeutet,
$R^8$ und $R^9$ für Wasserstoff stehen oder zusammen eine Bindung, Trimethylen, Tetramethylen, 2-Butenylen oder einen ankondensierten Benzolring bedeuten,
$R^{10}$ $C_{1-6}$-Alkyl und $R^{11}$ Trifluormethyl, $C_{1-6}$-Alkoxy, $C_{1-6}$-Alkoxy-$C_{1-4}$-alkyl, $C_{2-7}$-Alkanoyl, $C_{2-7}$-Alkoxycarbonyl oder $C_{2-7}$-Alkoxycarbonyl-$C_{1-4}$-alkyl bedeuten, oder $R^{10}$ und $R^{11}$ zusammen mit dem Kohlenstoffatom, an das sie geknüpft sind, einen Cyclopentanring bilden, der gegebenenfalls mit 1 bis 3 $C_{1-4}$-Alkyl und/oder 1 $C_{1-4}$-Alkoxy substituiert sein kann, wobei, falls $R^{10}$, $R^{11}$ und das Kohlenstoffatom einen gegebenenfalls substituierten Cyclopentanring bilden, $R^1$ Chlor, Brom oder Trifluormethyl ist,

$R^{12}$ Wasserstoff oder $C_{1-6}$-Alkyl und $R^{13}$ $C_{1-6}$-Alkyl; oder Phenyl, gegebenenfalls substituiert mit Halogen, $C_{1-4}$-Alkyl und/oder Nitro bedeuten, oder $R^{12}$ und $R^{13}$ zusammen mit dem Kohlenstoffatom, an das sie geknüpft sind, einen $C_{5-7}$-Cycloalkanring bilden, und

$R^{14}$ und $R^{15}$ $C_{1-6}$-Alkyl oder Phenyl bedeuten, und wobei, wenn $R^5$ eine Gruppe (a), (b) oder (c) darstellt, m die Zahl 1, n die Zahl 0 und p die Zahl 0 oder 1 bedeuten; wenn $R^5$ eine Gruppe (d) darstellt, m, n und p die Zahl 0 bedeuten; wenn $R^5$ eine Gruppe (e) darstellt, m die Zahl 0 und n und p die Zahl 1 bedeuten; und wenn $R^5$ eine Gruppe (f) darstellt, m und p die Zahl 0 oder 1 und n die Zahl 1 bedeuten,

sowie Formulierungshilfsstoffe enthält.

15. Unkrautbekämpfungsmittel nach Anspruch 14, dadurch gekennzeichnet, dass es eine wirksame Menge einer der in den Ansprüchen 5 bis 9 genannten Verbindungen enthält.

16. Verfahren zur Herstellung von Verbindungen der allgemeinen Formel

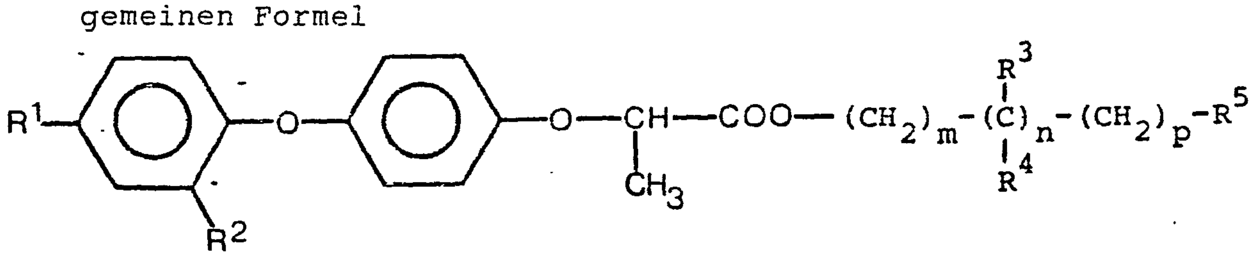

I

worin $R^1$ Chlor, Brom, Jod oder Trifluormethyl,

$R^2$ Wasserstoff, Chlor, Brom oder Jod,

$R^3$ Wasserstoff oder $C_{1-4}$-Alkyl,

$R^4$ Wasserstoff; $C_{1-4}$-Alkyl; oder Phenyl, gegebenenfalls substituiert mit Halogen, $C_{1-4}$-Alkyl und/oder Nitro,

wobei $R^3$ und $R^4$ zusammen mit dem Kohlenstoffatom, an das sie geknüpft sind, auch einen $C_{5-8}$-Cycloalkanring bilden können, der gegebenenfalls mit 1 bis 3 $C_{1-4}$-Alkyl-

gruppen substituiert sein kann,
m, n und p die Zahl 0 oder 1
und $R^5$ eine der folgenden Gruppen (a) - (f) bedeuten:

$-NHCO-R^6$ (a)

$-NHSO_2-R^7$ (b)

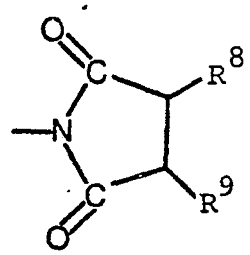
(c)

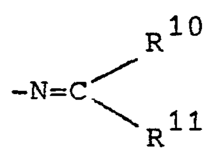
(d)

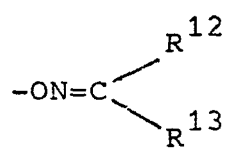
(e)

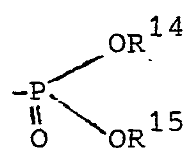
(f)

wobei in den obigen Formeln der Gruppen (a) - (f)

$R^6$ Wasserstoff; $C_{1-6}$-Alkyl; $C_{1-4}$-Halogenalkyl; oder Phenyl, gegebenenfalls substituiert mit Halogen, $C_{1-4}$-Alkyl und/oder Nitro bedeutet,

$R^7$ $C_{1-6}$-Alkyl; oder Phenyl, gegebenenfalls substituiert mit Halogen, $C_{1-4}$-Alkyl und/oder Nitro bedeutet,

$R^8$ und $R^9$ für Wasserstoff stehen oder zusammen eine Bindung, Trimethylen, Tetramethylen, 2-Butenylen oder einen ankondensierten Benzolring bedeuten,

$R^{10}$ $C_{1-6}$-Alkyl und $R^{11}$ Trifluormethyl, $C_{1-6}$-Alkoxy, $C_{1-6}$-Alkoxy-$C_{1-4}$-alkyl, $C_{2-7}$-Alkanoyl, $C_{2-7}$-Alkoxycarbonyl oder $C_{2-7}$-Alkoxycarbonyl-$C_{1-4}$-alkyl

bedeuten, oder $R^{10}$ und $R^{11}$ zusammen mit dem Kohlenstoffatom, an das sie geknüpft sind, einen Cyclopentanring bilden, der gegebenenfalls mit 1 bis 3 $C_{1-4}$-Alkyl und/oder 1 $C_{1-4}$-Alkoxy substituiert sein kann, wobei, falls $R^{10}$, $R^{11}$ und das Kohlenstoffatom einen gegebenenfalls substituierten Cyclopentanring bilden, $R^1$ Chlor, Brom oder Trifluormethyl ist,

$R^{12}$ Wasserstoff oder $C_{1-6}$-Alkyl und $R^{13}$ $C_{1-6}$-Alkyl; oder Phenyl, gegebenenfalls substituiert mit Halogen, $C_{1-4}$-Alkyl und/oder Nitro bedeuten, oder $R^{12}$ und $R^{13}$ zusammen mit dem Kohlenstoffatom, an das sie geknüpft sind, einen $C_{5-7}$-Cycloalkanring bilden, und

$R^{14}$ und $R^{15}$ $C_{1-6}$-Alkyl oder Phenyl bedeuten,

und wobei, wenn $R^5$ eine Gruppe (a), (b) oder (c) darstellt, m die Zahl 1, n die Zahl 0 und p die Zahl 0 oder 1 bedeuten; wenn $R^5$ eine Gruppe (d) darstellt, m, n und p die Zahl 0 bedeuten; wenn $R^5$ eine Gruppe (e) darstellt, m die Zahl 0 und n und p die Zahl 1 bedeuten; und wenn $R^5$ eine Gruppe (f) darstellt, m und p die Zahl 0 oder 1 und n die Zahl 1 bedeuten,

dadurch gekennzeichnet, dass man

a) eine Säure der Formel

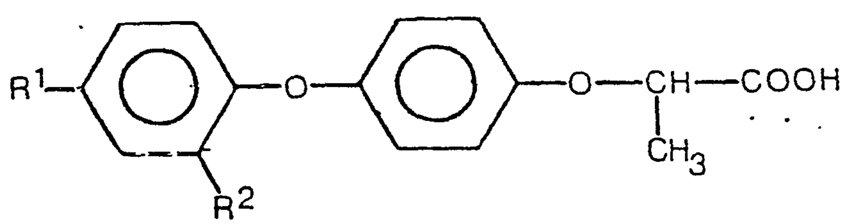

II

worin $R^1$ und $R^2$ die oben angegebenen Bedeutungen besitzen,

oder ein reaktionsfähiges Derivat davon, mit einer Verbindung der Formel

$$X-(CH_2)_m-(\overset{R^3}{\underset{R^4}{C}})_n-(CH_2)_p-R^5 \qquad III$$

worin $R^3$, $R^4$, $R^5$, m, n und p die oben angegebenen Bedeutungen besitzen und X eine Abgangsgruppe bedeutet, umsetzt, oder

b) ein Phenol der Formel

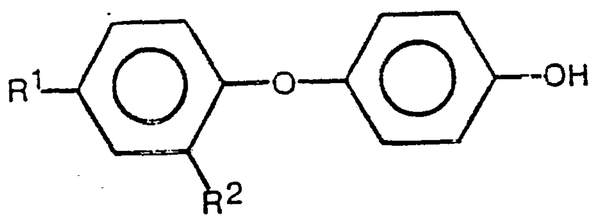

IV

worin $R^1$ und $R^2$ die oben angegebenen Bedeutungen besitzen,

oder ein Alkalimetallsalz davon, mit einer Verbindung der Formel

$$Y-CH(CH_3)-COO-(CH_2)_m-(C(R^3)(R^4))_n-(CH_2)_p-R^5 \quad V$$

worin $R^3$, $R^4$, $R^5$, m, n und p die oben angegebenen Bedeutungen besitzen und Y eine Abgangsgruppe bedeutet, umsetzt.

17. Verfahren zur Bekämpfung von Unkräutern, dadurch gekennzeichnet, dass man das gegen Unkräuter zu schützende Gut und/oder die Unkräuter mit einer wirksamen Menge einer der in den Ansprüchen 1 bis 10 genannten Verbindungen bzw. eines in Anspruch 14 oder 15 genannten Mittels behandelt.

18. Verfahren zur Bekämpfung von Unkräutern, dadurch gekennzeichnet, dass man das gegen Unkräuter zu schützende Gut und/oder die Unkräuter mit einer wirksamen Menge einer der in Anspruch 11 genannten Verbindungen behandelt.

19. Verwendung einer der in den Ansprüchen 1 bis 10 genannten Verbindungen bzw. eines in Anspruch 14 oder 15 genannten Mittels zur Bekämpfung von Unkräutern.

20. Verwendung einer der in Anspruch 11 genannten Verbindungen zur Bekämpfung von Unkräutern.

***

## Patentansprüche
für OESTERREICH

1. Unkrautbekämpfungsmittel, dadurch gekennzeichnet, dass es eine wirksame Menge mindestens einer Verbindung der allgemeinen Formel

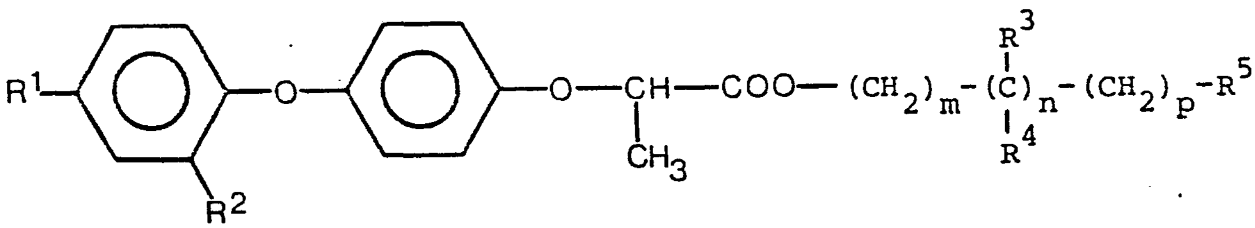

I

worin $R^1$ Chlor, Brom, Jod oder Trifluormethyl,
$R^2$ Wasserstoff, Chlor, Brom oder Jod,
$R^3$ Wasserstoff oder $C_{1-4}$-Alkyl,
$R^4$ Wasserstoff; $C_{1-4}$-Alkyl; oder Phenyl, gegebenenfalls substituiert mit Halogen, $C_{1-4}$-Alkyl und/oder Nitro,
wobei $R^3$ und $R^4$ zusammen mit dem Kohlenstoffatom, an das sie geknüpft sind, auch einen $C_{5-8}$-Cycloalkanring bilden können, der gegebenenfalls mit 1 bis 3 $C_{1-4}$-Alkylgruppen substituiert sein kann,
m, n und p die Zahl 0 oder 1
und $R^5$ eine der folgenden Gruppen (a) - (f) bedeuten:

$-NHCO-R^6$ (a)

$-NHSO_2-R^7$ (b)

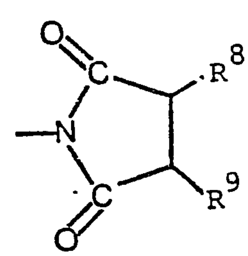

(c)

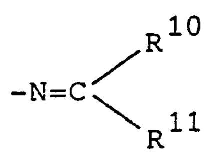

(d)

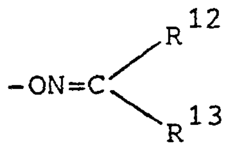

(e)

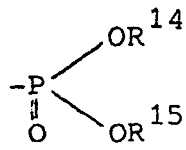

(f)

wobei in den obigen Formeln der Gruppen (a) - (f) $R^6$ Wasserstoff; $C_{1-6}$-Alkyl; $C_{1-4}$-Halogenalkyl; oder Phenyl, gegebenenfalls substituiert mit Halogen, $C_{1-4}$-Alkyl und/oder Nitro bedeutet,
$R^7$ $C_{1-6}$-Alkyl; oder Phenyl, gegebenenfalls substituiert mit Halogen, $C_{1-4}$-Alkyl und/oder Nitro bedeutet,
$R^8$ und $R^9$ für Wasserstoff stehen oder zusammen eine Bindung, Trimethylen, Tetramethylen, 2-Butenylen oder einen ankondensierten Benzolring bedeuten,
$R^{10}$ $C_{1-6}$-Alkyl und $R^{11}$ Trifluormethyl, $C_{1-6}$-Alkoxy, $C_{1-6}$-Alkoxy-$C_{1-4}$-alkyl, $C_{2-7}$-Alkanoyl, $C_{2-7}$-Alkoxycarbonyl oder $C_{2-7}$-Alkoxycarbonyl-$C_{1-4}$-alkyl bedeuten, oder $R^{10}$ und $R^{11}$ zusammen mit dem Kohlenstoffatom, an das sie geknüpft sind, einen Cyclopentanring bilden, der gegebenenfalls mit 1 bis 3 $C_{1-4}$-Alkyl und/oder 1 $C_{1-4}$-Alkoxy substituiert sein kann, wobei, falls $R^{10}$, $R^{11}$ und das Kohlenstoffatom einen gegebenenfalls substituierten Cyclopentanring bilden, $R^1$ Chlor, Brom oder Trifluormethyl ist,
$R^{12}$ Wasserstoff oder $C_{1-6}$-Alkyl und $R^{13}$ $C_{1-6}$-Alkyl; oder Phenyl, gegebenenfalls substituiert mit Halogen, $C_{1-4}$-Alkyl und/oder Nitro bedeuten, oder $R^{12}$ und $R^{13}$ zusammen mit dem Kohlenstoffatom, an das sie geknüpft sind, einen $C_{5-7}$-Cycloalkanring bilden, und
$R^{14}$ und $R^{15}$ $C_{1-6}$-Alkyl oder Phenyl bedeuten,
und wobei, wenn $R^5$ eine Gruppe (a), (b) oder (c) darstellt, m die Zahl 1, n die Zahl 0 und p die Zahl 0 oder 1 bedeuten; wenn $R^5$ eine Gruppe (d) darstellt, m, n und p die Zahl 0 bedeuten; wenn $R^5$ eine Gruppe

(e) darstellt, m die Zahl 0 und n und p die Zahl 1 bedeuten; und wenn $R^5$ eine Gruppe (f) darstellt, m und p die Zahl 0 oder 1 und n die Zahl 1 bedeuten, sowie Formulierungshilfsstoffe enthält.

2. Unkrautbekämpfungsmittel nach Anspruch 1, worin $R^1$ in der Formel I Jod oder Trifluormethyl bedeutet.

3. Unkrautbekämpfungsmittel nach Anspruch 1 oder 2, worin $R^2$ in der Formel I Wasserstoff oder Chlor bedeutet.

4. Unkrautbekämpfungsmittel nach einem der Ansprüche 1 bis 3, worin als Verbindung bzw. Verbindungen der Formel I die D-Isomeren verwendet werden.

5. Unkrautbekämpfungsmittel nach Anspruch 1, dadurch gekennzeichnet, dass es eine wirksame Menge von D-2-[p-(α,α,α-Trifluor-p-tolyloxy)phenoxy]-propionsäure-succinimidomethylester sowie Formulierungshilfsstoffe enthält.

6. Unkrautbekämpfungsmittel nach Anspruch 1, dadurch gekennzeichnet, dass es eine wirksame Menge von Aethyl-N-/D-2-[p-(α,α,α- trifluor-p-tolyloxy)phenoxy]-propionoxy/-acetimidat sowie Formulierungshilfsstoffe enthält.

7. Unkrautbekämpfungsmittel nach Anspruch 1, dadurch gekennzeichnet, dass es eine wirksame Menge von n-Butyl-N-/D-2-[p-(α,α,α-trifluor-p-tolyloxy)phenoxy]-propionoxy/-acetimidat sowie Formulierungshilfsstoffe enthält.

8. Unkrautbekämpfungsmittel nach Anspruch 1, dadurch gekennzeichnet, dass es eine wirksame Menge von D-2-[p-(α,α,α-Trifluor-p-tolyloxy)phenoxy]-propionsäure-2-isopropylidenaminooxy-äthylester sowie Formulierungshilfsstoffe enthält.

9. Unkrautbekämpfungsmittel nach Anspruch 1, dadurch gekennzeichnet, dass es eine wirksame Menge von D-2-[p-(α,α,α-Trifluor-p-tolyloxy)phenoxy]-propionsäure-diäthoxyphosphinylmethylester sowie Formulierungshilfsstoffe enthält.

10. Unkrautbekämpfungsmittel nach Anspruch 1, dadurch gekennzeichnet, dass es eine wirksame Menge mindestens einer Verbindung ausgewählt unter:

D-2-[p-(α,α,α-Trifluor-p-tolyloxy)phenoxy]-propionsäure-acetamidomethylester,

D-2-[p-(α,α,α-Trifluor-p-tolyloxy)phenoxy]-propionsäure-chloracetamidomethylester,

D-2-[p-(α,α,α-Trifluor-p-tolyloxy)phenoxy]-propionsäure-trichloracetamidomethylester,

D-2-[p-(α,α,α-Trifluor-p-tolyloxy)phenoxy]-propionsäure-2-methansulfonamido-äthylester,

D-2-[p-(α,α,α-Trifluor-p-tolyloxy)phenoxy]-propionsäure-phthalimidomethylester,

D-2-[p-(α,α,α-Trifluor-p-tolyloxy)phenoxy]-propionsäure-2-diäthoxyphosphinyl-äthylester,

D-2-[p-(α,α,α-Trifluor-p-tolyloxy)phenoxy]-propionsäure-1-diäthoxyphosphinyl-äthylester,

D-2-[p-(α,α,α-Trifluor-p-tolyloxy)phenoxy]-propionsäure-1-diäthoxyphosphinyl-n-butylester,

D-2-[p-(α,α,α-Trifluor-p-tolyloxy)phenoxy]-propionsäure-α-diäthoxyphosphinyl-benzylester,

3-[D-2-[p-(α,α,α-Trifluor-p-tolyloxy)phenoxy]-propionoxyimino]-buttersäure-äthylester,

D-2-[p-(α,α,α-Trifluor-p-tolyloxy)phenoxy]-propionsäure-1,1,1-trifluoracetonoximester,

Aethyl-N-[D-2-[p-(α,α,α-trifluor-p-tolyloxy)phenoxy]-propionoxy]-n-butyrimidat,

D-2-[p-(α,α,α-Trifluor-p-tolyloxy)phenoxy]-propionsäure-methoxyacetonoximester,

D-2-[p-(α,α,α-Trifluor-p-tolyloxy)phenoxy]-propionsäure-diacetonmonoximester,

2-⁄D-2-[p-(α,α,α-Trifluor-p-tolyloxy)phenoxy]-propionoxyimino⁄-propionsäure-äthylester,

2-Methyl-3-⁄D-2-[p-(α,α,α-trifluor-p-tolyloxy)-phenoxy]-propionoxyimino⁄-buttersäure-äthylester,

4-⁄D-2-[p-(α,α,α-Trifluor-p-tolyloxy)phenoxy]-propionoxyimino⁄-n-valeriansäure-äthylester,

D-2-[p-(o-Chlor-p-trifluormethyl-phenoxy)phenoxy]-propionsäure-2-isopropylidenaminooxy-äthylester,

D-2-[p-(α,α,α-Trifluor-p-tolyloxy)phenoxy]-propionsäure-cyclopentanonoximester,

D-2-[p-(α,α,α-Trifluor-p-tolyloxy)phenoxy]-propionsäure-1-diäthoxyphosphinyl-cyclohexylester,

D-2-[p-(α,α,α-Trifluor-p-tolyloxy)phenoxy]-propionsäure-2-dimethoxyphosphinyl-2-propylester und

D-2-[p-(α,α,α-Trifluor-p-tolyloxy)phenoxy]-propionsäure-3-diäthoxyphosphinyl-propylester,

sowie Formulierungshilfsstoffe enthält.

11. Unkrautbekämpfungsmittel nach Anspruch 1, dadurch gekennzeichnet, dass es eine wirksame Menge mindestens einer Verbindung ausgewählt unter:

D-2-[p-(p-Jodphenoxy)phenoxy]-propionsäure-2-isopropylidenaminooxy-äthylester,

D-2-[p-(p-Jodphenoxy)phenoxy]-propionsäure-2-methansulfonamido-äthylester,

D-2-[p-(p-Jodphenoxy)phenoxy]-propionsäure-succinimidomethylester,

D-2-[p-(p-Jodphenoxy)phenoxy]-propionsäure-diäthoxyphosphinylmethylester,

D-2-[p-(p-Jodphenoxy)phenoxy]-propionsäure-2-diäthoxyphosphinyl-äthylester,

D-2-[p-(o-Chlor-p-jodphenoxy)phenoxy]-propionsäure-2-isopropylidenaminooxy-äthylester,

D-2-[p-(o-Chlor-p-jodphenoxy)phenoxy]-propionsäure-2-methansulfonamido-äthylester,

D-2-[p-(o-Chlor-p-jodphenoxy)phenoxy]-propionsäure-diäthoxyphosphinylmethylester,

D-2-[p-(α,α,α-Trifluor-p-tolyloxy)phenoxy]-propionsäure-dibutoxyphosphinylmethylester,

D-2-[p-(α,α,α-Trifluor-p-tolyloxy)phenoxy]-propionsäure-1-diphenoxyphosphinyl-äthylester,

D-2-[p-(α,α,α-Trifluor-p-tolyloxy)phenoxy]-propionsäure-1-isopropylidenaminooxy-2-propylester,

D-2-[p-(α,α,α-Trifluor-p-tolyloxy)phenoxy]-propionsäure-1-isopropylidenaminooxy-2-butylester,

D-2-[p-(α,α,α-Trifluor-p-tolyloxy)phenoxy]-propionsäure-1-isopropylidenaminooxy-1-phenyläthylester,

D-2-[p-(α,α,α-Trifluor-p-tolyloxy)phenoxy]-propionsäure-2-(2-butylidenaminooxy)-äthylester,

D-2-[p-(α,α,α-Trifluor-p-tolyloxy)phenoxy]-propionsäure-2-(α-methylbenzylidenaminooxy)-äthylester,

D-2-[p-(α,α,α-Trifluor-p-tolyloxy)phenoxy]-propionsäure-2-cyclohexylidenaminooxy-äthylester und

D-2-[p-(p-Jodphenoxy)phenoxy]-propionsäure-3-diäthoxyphosphinyl-propylester,

sowie Formulierungshilfsstoffe enthält.

12. Verfahren zur Herstellung von Verbindungen der allgemeinen Formel

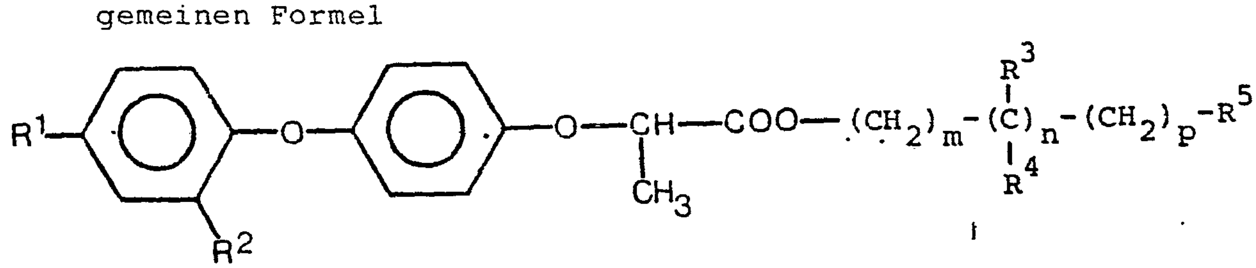

I

worin $R^1$ Chlor, Brom, Jod oder Trifluormethyl,

$R^2$ Wasserstoff, Chlor, Brom oder Jod,

$R^3$ Wasserstoff oder $C_{1-4}$-Alkyl,

$R^4$ Wasserstoff; $C_{1-4}$-Alkyl; oder Phenyl, gegebenenfalls substituiert mit Halogen, $C_{1-4}$-Alkyl und/oder Nitro,

wobei $R^3$ und $R^4$ zusammen mit dem Kohlenstoffatom, an das sie geknüpft sind, auch einen $C_{5-8}$-Cycloalkanring bilden können, der gegebenenfalls mit 1 bis 3 $C_{1-4}$-Alkylgruppen substituiert sein kann,

m, n und p die Zahl 0 oder 1

und $R^5$ eine der folgenden Gruppen (a) - (f) bedeuten:

$-NHCO-R^6$ (a)

$-NHSO_2-R^7$ (b)

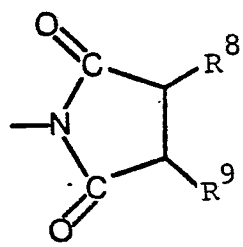 (c)

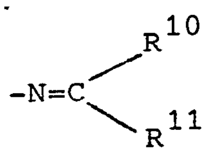 (d)

m, n und p die Zahl 0 bedeuten; wenn $R^5$ eine Gruppe (e) darstellt, m die Zahl 0 und n und p die Zahl 1 bedeuten; und wenn $R^5$ eine Gruppe (f) darstellt, m und p die Zahl 0 oder 1 und n die Zahl 1 bedeuten, dadurch gekennzeichnet, dass man

a) eine Säure der Formel

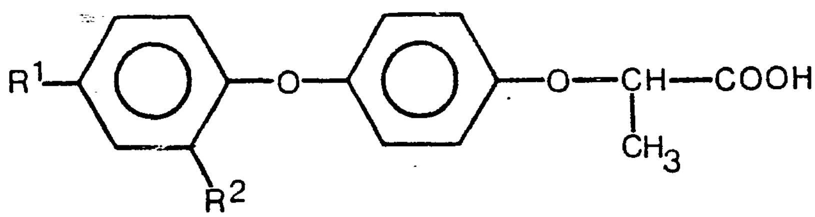

II

worin $R^1$ und $R^2$ die oben angegebenen Bedeutungen besitzen,

oder ein reaktionsfähiges Derivat davon, mit einer Verbindung der Formel

$$X-(CH_2)_m-(\overset{R^3}{\underset{R^4}{C}})_n-(CH_2)_p-R^5 \qquad III$$

worin $R^3$, $R^4$, $R^5$, m, n und p die oben angegebenen Bedeutungen besitzen und X eine Abgangsgruppe bedeutet, umsetzt, oder

b) ein Phenol der Formel

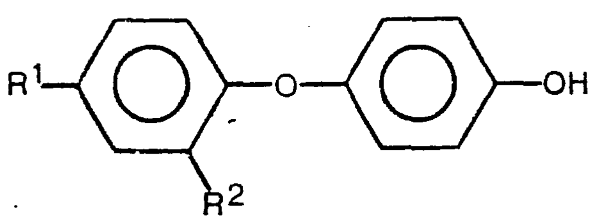

IV

worin $R^1$ und $R^2$ die oben angegebenen Bedeutungen besitzen,

oder ein Alkalimetallsalz davon, mit einer Verbindung der Formel

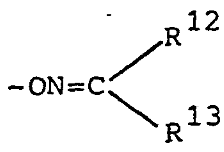 (e)

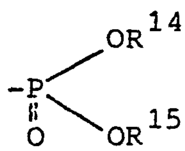 (f)

wobei in den obigen Formeln der Gruppen (a) - (f)

$R^6$ Wasserstoff; $C_{1-6}$-Alkyl; $C_{1-4}$-Halogenalkyl; oder Phenyl, gegebenenfalls substituiert mit Halogen, $C_{1-4}$-Alkyl und/oder Nitro bedeutet,

$R^7$ $C_{1-6}$-Alkyl; oder Phenyl, gegebenenfalls substituiert mit Halogen, $C_{1-4}$-Alkyl und/oder Nitro bedeutet,

$R^8$ und $R^9$ für Wasserstoff stehen oder zusammen eine Bindung, Trimethylen, Tetramethylen, 2-Butenylen oder einen ankondensierten Benzolring bedeuten,

$R^{10}$ $C_{1-6}$-Alkyl und $R^{11}$ Trifluormethyl, $C_{1-6}$-Alkoxy, $C_{1-6}$-Alkoxy-$C_{1-4}$-alkyl, $C_{2-7}$-Alkanoyl, $C_{2-7}$-Alkoxycarbonyl oder $C_{2-7}$-Alkoxycarbonyl-$C_{1-4}$-alkyl bedeuten, oder $R^{10}$ und $R^{11}$ zusammen mit dem Kohlenstoffatom, an das sie geknüpft sind, einen Cyclopentanring bilden, der gegebenenfalls mit 1 bis 3 $C_{1-4}$-Alkyl und/oder 1 $C_{1-4}$-Alkoxy substituiert sein kann, wobei, falls $R^{10}$, $R^{11}$ und das Kohlenstoffatom einen gegebenenfalls substituierten Cyclopentanring bilden, $R^1$ Chlor, Brom oder Trifluormethyl ist,

$R^{12}$ Wasserstoff oder $C_{1-6}$-Alkyl und $R^{13}$ $C_{1-6}$-Alkyl; oder Phenyl, gegebenenfalls substituiert mit Halogen, $C_{1-4}$-Alkyl und/oder Nitro bedeuten, oder $R^{12}$ und $R^{13}$ zusammen mit dem Kohlenstoffatom, an das sie geknüpft sind, einen $C_{5-7}$-Cycloalkanring bilden, und

$R^{14}$ und $R^{15}$ $C_{1-6}$-Alkyl oder Phenyl bedeuten,

und wobei, wenn $R^5$ eine Gruppe (a), (b) oder (c) darstellt, m die Zahl 1, n die Zahl 0 und p die Zahl 0 oder 1 bedeuten; wenn $R^5$ eine Gruppe (d) darstellt,

EP 81 10 9203.0

1. Patentansprüche: 1-4, 10-20 zum Teil, 5

Propionsäureester, die Amido-, Imido- oder Sulfonamidogruppen aufweisen, Verfahren zu deren Herstellung, Unkrautbekämpfungsmittel, die diese Verbindungen als Wirkstoffe enthalten, sowie Verwendung solcher Verbindungen und Mittel zur Bekämpfung von Unkräutern

2. Patentansprüche: 1-4, 10-20 zum Teil, 6-8

Oximether und Oximester der Formel I, sowie Hydroxylaminoester der Formel I, Verfahren zu deren Herstellung, Unkrautbekämpfungsmittel, die diese Verbindungen als Wirkstoffe enthalten, sowie Verwendung solcher Verbindungen und Mittel zur Bekämpfung von Unkräutern

3. Patentansprüche: 1-4, 10-20 zum Teil, 9

Phosphonsäureester der Formel I, Verfahren zu deren Herstellung, Unkrautbekämpfungsmittel, die diese Verbindungen enthalten, sowie Verwendung solcher Verbindungen und Mittel zur Bekämpfung von Unkräutern

------